(19) Europäisches Patentamt
European Patent Office
Office européen des brevets

(11) Veröffentlichungsnummer: **0 169 327**
**B1**

(12) # EUROPÄISCHE PATENTSCHRIFT

(45) Veröffentlichungstag der Patentschrift: **13.01.88**

(21) Anmeldenummer: **85106174.7**

(22) Anmeldetag: **20.05.85**

(51) Int. Cl.⁴: **C 07 C 161/04,** C 07 D 239/26, C 07 D 319/06, C 09 K 19/34, C 09 K 19/30

(54) **Phenylisothiocyanate.**

(30) Priorität: **25.05.84 CH 2571/84**
**12.03.85 CH 1113/85**

(43) Veröffentlichungstag der Anmeldung: **29.01.86 Patentblatt 86/5**

(45) Bekanntmachung des Hinweises auf die Patenterteilung: **13.01.88 Patentblatt 88/2**

(84) Benannte Vertragsstaaten: **AT BE CH DE FR GB IT LI NL SE**

(56) Entgegenhaltungen:
**EP-A-0 126 883**

**MOLECULAR CRYSTALS AND LIQUID CRYSTALS, Band 87, Nr. 1,2, 1982, Seiten 109-135, Gordon and Breach Science Publishers Inc., New York, US; R. DABROWSKI et al.: "Mesomorphic properties of 4-n-pentylbiphenyl derivatives"**
**MOLECULAR CRYSTALS AND LIQUID CRYSTALS, Band 124, Nr. 1/4, März 1985, Seiten 241-257, Gordon and Breach, Science Publishers, Inc. and OPA Ltd., New York, US; R. DABROWSKI et al.: "Mesomorphic characteristics of some new homologous series with the isothiocyanato terminal group"**

(73) Patentinhaber: **F. HOFFMANN- LA ROCHE & CO. Aktiengesellschaft, CH- 4002 Basel (CH)**

(72) Erfinder: **Petrzilka, Martin, Dr., Schwarzackerstrasse 54, CH- 4303 Kaiseraugst (CH)**
Erfinder: **Schadt, Martin, Dr., Liestalerstrasse 77, CH- 4411 Seltisberg (CH)**
Erfinder: **Villiger, Alois, Dr., Im Ettingerhof 5, CH- 4055 Basel (CH)**

(74) Vertreter: **Lederer, Franz, Dr., Van der Werth, Lederer & Riederer Patentanwälte Lucile- Grahn- Strasse 22, D-8000 München 80 (DE)**

**EP 0 169 327 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue Phenylisothiocyanate und deren Herstellung sowie flüssigkristalline Gemische, welche diese Verbindungen enthalten, und die Verwendung der Verbindungen und Mischungen für elektro-optische Zwecke.

Flüssige Kristalle haben in den letzten Jahren vor allem als Dielektrika in Anzeigevorrichtungen stark an Bedeutung gewonnen, da die optischen Eigenschaften solcher Substanzen durch eine angelegte Spannung beeinflusst werden können. Elektro-optische Vorrichtungen auf der Basis von Flussigkristallen sind dem Fachmann gut bekannt und können auf verschiedenen Effekten beruhen, wie beispielsweise der dynamischen Streuung, der Deformation aufgerichteter Phasen (DAP-Typ), dem Schadt-Helfrich-Effekt (Drehzelle), dem Gast/Wirt-Effekt (Guest/Host-Effekt) oder einem cholesterisch-nematischen Phasenübergang (Phase-Change-Effekt).

Damit Flüssigkristalle als Dielektrika für elektro-optische Anzeigevorrichtungen geeignet sind, müssen sie jedoch einer Reihe von Anforderungen genügen. Beispielsweise müssen sie eine gute chemische Stabilität gegenüber Umwelteinflüssen, wie z. B. Wärme, Feuchtigkeit, Luft und elektromagnetische Strahlung im infraroten, sichtbaren und ultravioletten Bereich besitzen. Ferner sollten sie farblos sein, kurze Ansprechzeiten und nicht zu hohe Viskosität aufweisen, einen guten Kontrast ergeben und im ganzen Temperaturbereich, in welchem dis Flüssigkristallzelle betrieben werden soll, eine nematische bzw. cholesterische Mesophase besitzen. Weitere Eigenschaften müssen je nach verwendetem Zellentyp unterschiedliche Bedingungen erfüllen, beispielsweise sollten Flüssigkristalls, die in Drehzellen verwendet werden, eine grosse positive Anisotropie der Dielektrizitätskonstante ($\Delta\varepsilon = \varepsilon_{\parallel} - \varepsilon_{\perp} > 0$, wobei $\varepsilon_{\parallel}$ die Dielektrizitätskonstante entlang der Moleküllängsachse und $\varepsilon_{\perp}$ die Dielektrizitätskonstante senkrecht dazu bedeuten), und Flüssigkristalle, die in Guest/Host-Zellen verwendet werden, eine grosse positive oder negative Anisotropie der Dielektrizitätskonstante aufweisen. In beiden Fällen ist zudem eine niedrige Schwellenspannung und eine möglichst geringe Leitfähigkeit erwünscht.

Da es im allgemeinen nicht möglich ist alle gewünschten und zum Teil widersprüchlichen Eigenschaften mit einer einzigen Verbindung zu erreichen, wird meist versucht, durch Mischen mehrerer Komponenten die Eigenschaften für die jeweiligen Anwendungsn zu optimieren. Hierbei ist jedoch wichtig, dass die Komponenten untereinander keine chemischen Reaktionen eingehen und gut mischbar sind. Ferner sollten die gebildeten Mischungen mindestens bei Temperaturen, bei denen die Flüssigkristallzelle betrieben werden soll, keine smektischen Mesophasen aufweissn.

Es wurde nun gefunden, dass die Phenylisothiocyanate der allgemeinen Formel

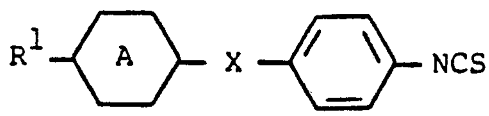

I

worin X eine einfache Kovalenzbindung, die Äthylengruppe $-CH_2CH_2-$, 1,4-Phenylen, einen 2,5-disubstituierten Pyrimidinring oder die Gruppe der Formel

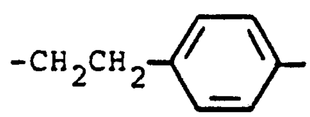

IA

darstellt; Ring A trans-1,4-Cyclohexylen oder, sofern X eine einfache Kovalenzbindung darstellt, auch 1,4-Phenylen, einen 2,5-disubstituierten Pyrimidinring oder einen trans-2,5-disubstituierten m-Dioxanring bezeichnet; und R geradkettiges trans-1-Alkenyl mit 2 bis 12 Kohlenstoffatomen oder geradkettiges trans-3-Alkenyl mit 4 bis 12 Kohlenstoffatomen bedeutet oder, sofern X $-CH_2CH_2-$, 1,4-Phenylen, einen 2,5-disubstituierten Pyrimidinring oder eine Gruppe der Formel IA darstellt odsr Ring A sinen 2,5-disubstituierten Pyrimidinring oder einen trans-2,5-disubstituierten m-Dioxanring bezeichnet, $R^1$ auch geradkettiges Alkyl mit 1 bis 12 Kohlenstoffatomen bedeutet; und einer der in Formel I vorhandenen Benzolringe gegebenenfalls einen lateralen Fluor-Substituenten aufweist,

die geforderten Eigenschaften aufweisen.

Insbesondere ergeben die erfindungsgemässen Verbindungen der Formel I bei Anwendung in elektrooptischen Vorrichtungen steile Transmissionskurven und kurze Schaltzeiten. Die erfindungsgemässen Phenylisothiocyanate besitzen zudem niedere Viskosität und eine gute chemische Stabilität. Die Klärpunkte liegen häufig günstiger als diejenigen vergleichbarer bekannter Flüssigkristallkomponenten. Die VVerbindungen mit lateralem Fluor an einem Benzolring (d.h. mit einer 2-Fluor-1,4-phenylengruppe) zeigen im allgemeinen verbesserte nematische Tendenzen und leicht modifizierte dielektrische Anisotropien. Die Verbindungen der Formel I sind gut mischbar mit bekannten Flüssigkristallen und können grundsätzlich in beliebigen Flüssigkristallen verwendet werden. Aufgrund ihrer grossen positiven Anisotropie der Dielektrizitätskonstante eignen sie sich jedoch insbesondere zur Anwendung in Drehzellen und Gast/Wirts-Zellen.

Im Rahmen der vorliegenden Erfindung umfasst der obige Ausdruck "geradkettiges trans-1-Alkenyl" die Gruppen Vinyl, trans-1-Propenyl, trans-1-Butenyl, trans-1-Pentenyl, trans-1-Hexenyl, trans-1-Heptenyl, trans-1-Octenyl, trans-1-Nonenyl, trans-1-Decenyl, trans-1-Undecenyl und trans-1-Dodecenyl. Der Ausdruck "geradkettiges trans-3-Alkenyl" umfasst die Gruppen 3-Butenyl, trans-3-Pentenyl, trans-3-Hexenyl, trans-3-Heptenyl, trans-3-Octenyl, trans-3-Nonenyl, trans-3-Decenyl, trans-3-Undecenyl und trans-3-Dodecenyl. Der Ausdruck "geradkettiges Alkyl" umfasst die Gruppen Methyl, Äthyl, Propyl, Butyl, Pentyl, Hexyl, Heptyl, Octyl, Nonyl, Decyl, Undecyl und Dodecyl.

Ein gegebenenfalls in obiger Formel I vorhandener Pyrimidinring oder m-Dioxanring kann in 2-Stellung oder 5-Stellung mit dem Benzolring verknüpft sein. Steht Ring A in obiger Formel I für einen 2,5-disubstituierten Pyrimidinring, so bedeutet $R^1$ vorzugsweise geradkettiges Alkyl mit 1 bis 12 Kohlenstoffatomen oder geradkettiges trans-3-Alkenyl mit 4 bis 12 Kohlenstoffatomen. Steht Ring A für 1,4-Phenylen, so bedeutet $R^1$ vorzugsweise geradkettiges trans-3-Alkenyl mit 4 bis 12 Kohlenstoffstomen. Ring A in obiger Formel I steht vorzugswsise für einen Cyclohexan-, Pyrimidin- oder Dioxanring. Ein gegebenenfalls vorhandener, lateraler Fluor-Substituent steht vorzugsweise in ortho-Stellung zur NCS-Gruppe. Besonders bevorzugt sind jedoch Verbindungen ohne Fluor-Substituenten.

In obiger Formel I steht X vorzugsweise für eine einfache Kovalenzbindung, für 1,4-Phenylen oder für einen 2,5-disubstituierten Pyrimidinring. Besonders bevorzugt sind diejenigen Verbindungen. worin X eine einfache Kovalenzbindung darstellt.

Bevorzugte Reste $R^1$ sind geradkettiges trans-1-Alkenyl mit 2 bis 7 Kohlenstoffatomen, geradkettiges trans-3-Alkenyl mit 4 bis 7 Kohlenstoffatomen und geradkettiges Alkyl mit 1 bis 7, insbesondere 2 bis 5 Kohlenstoffatomen.

Bevorzugte Gruppen erfindungsgemässer Verbindungen sind die Verbindungen der allgemeinen Formeln

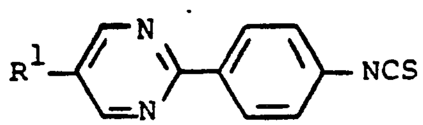
Ia

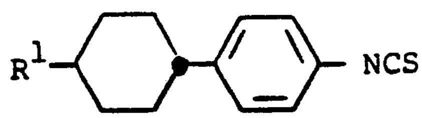
Ib

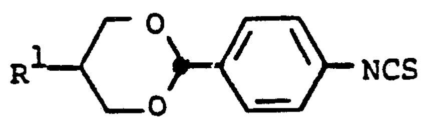
Ic

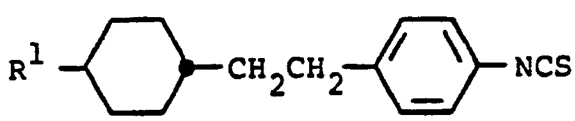
Id

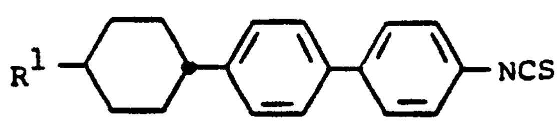
Ie

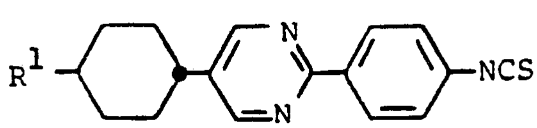
If

worin $R^1$ jeweils die obigen Bedeutungen hat.

Die Verbindungen der Formel I können erfindungsgemäss dadurch hergestellt werden, dass man ein Phenyldithiocarbamat der allgemeinen Formel

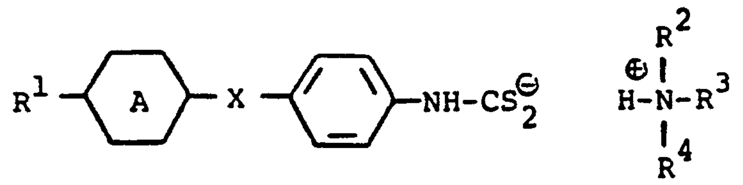

II

worin $R^1$, X und Ring A die obigen Bedeutungen haben, einer der Benzolringe gegebenenfalls einen lateralen Fluor-Substituenten aufweist, $R^2$ und $R^3$ $C_1$-$C_7$ -Alkyl bezeichnen und $R^4$ Wasserstoff oder $C_1$-$C_7$-Alkyl bedeutet, in Gegenwart eines Amins mit einem Chlorameisensäureester umsetzt.

Die Umsetzung der Verbindungen der Formel II kann in an sich bekannter Weise erfolgen. Bevorzugte Amine sind Dialkylamine und insbesondere Trialkylamine, worin "Alkyl" jeweils für Gruppen mit 1 bis 7 Kohlenstoffatomen steht. Besonders bevorzugt ist Triäthylamin. Bevorzugte Chlorameisensäureester sind die Chlorameisensäurealkylester, worin der Alkylteil 1 bis 7 Kohlenstoffatomen aufweist, wie Chlorameisensäuremethylester und Chlorameisensäureäthylester. Die Reaktion wird zweckmässig in einem polaren organischen Lösungsmittel durchgeführt, beispielsweise einem Äther, chlorierten Kohlenwasserstoff oder Nitril, wie Tetrahydrofuran, Dioxan, Methylenchlorid, Chloroform oder Acetonitril. Temperatur und Druck sind nicht kritisch. Vorzugsweise wird jedoch bei Atmosphärendruck und einer Temperatur von etwa 0° bis 40° C gearbeitet.

Die Verbindungen der Formel II sind neu. Sie können beispielsweise nach folgendem Reaktionsschema 1 hergestellt werden. worin $R^1$, $R^2$, $R^3$, $R^4$, X und Ring A die obigen Bedeutungen haben und jeweils einer der Benzolringe gegebenenfalls einen lateralen Fluor-Substituenten aufweist:

**Schema 1**

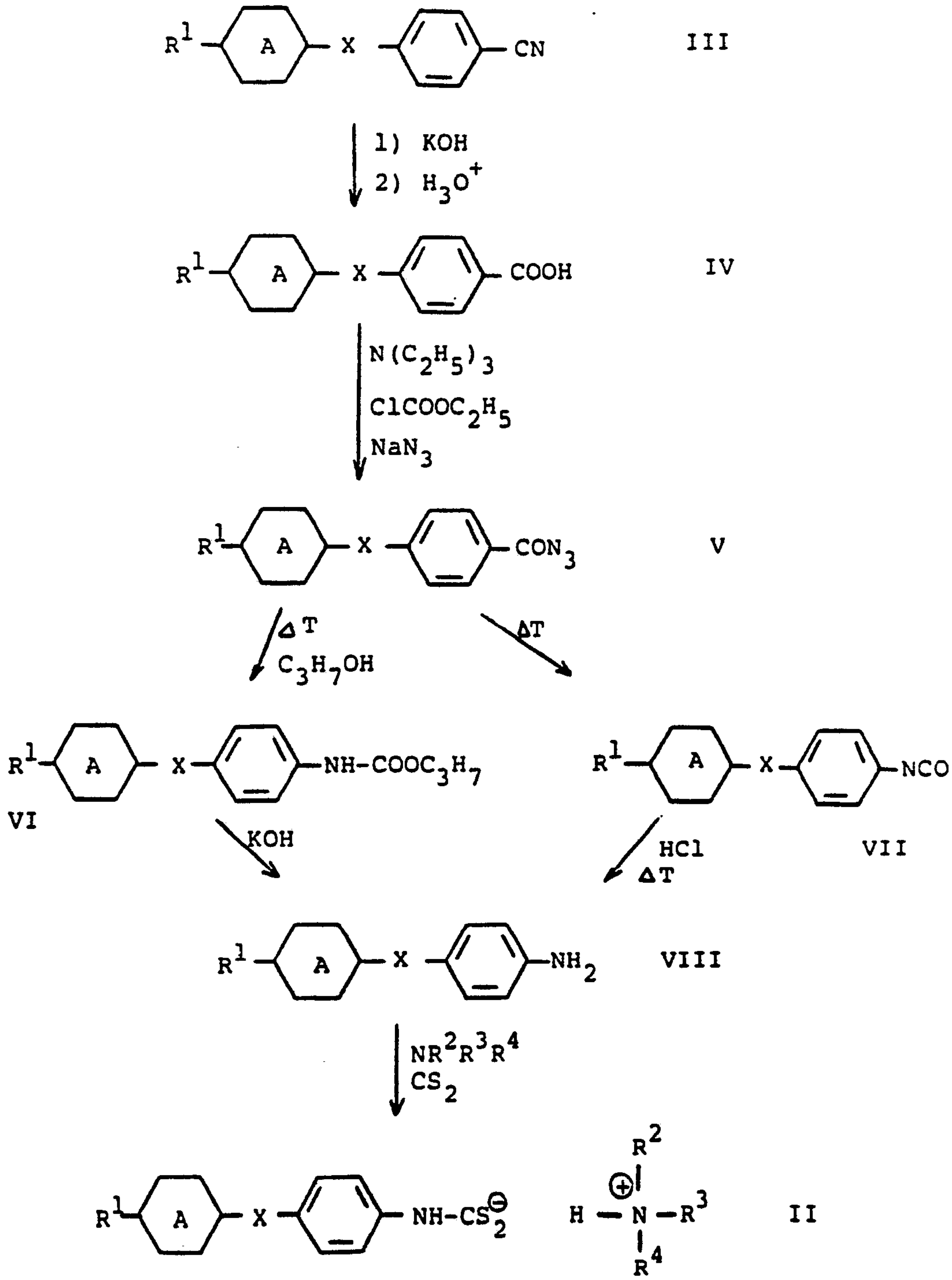


Enthält die Verbindung der Formel V sine Alkenylgruppe oder einen Dioxanring, so erfolgt die weitere Umsetzung zweckmässig via Verbindung der Formel VI.

Die Verbindungen der Formeln III und IV, worin $R^1$ geradkettiges Alkyl bedeutet, sind bekannte oder Analoge bekannter Verbindungen.

Die Verbindungen der Formel III, worin $R^1$ geradkettiges trans-1-Alkenyl oder geradkettiges trans-3-Alkenyl bedeutet, sind neu und besitzen ebenfalls flüssigkristalline Eigenschaften. Die Herstellung dieser Verbindungen kann nach an sich bekannten Methoden erfolgen und wird anhand repräsentativer Beispiele in

den folgenden Reaktionsschemata 2-7 näher veranschaulicht, worin $R^5$ Wasserstoff oder geradkettiges $C_1$-$C_{10}$-Alkyl, $R^6$ geradkettiges $C_1$-$C_{10}$-Alkyl, $R^7$ geradkettiges $C_1$-$C_9$-Alkyl, $R^8$ Wasserstoff oder geradkettiges $C_1$-$C_8$-Alkyl, $R^9$ geradkettiges $C_1$-$C_8$-Alkyl und Ts p-Tosyl bedeuten.

**Schema 2**

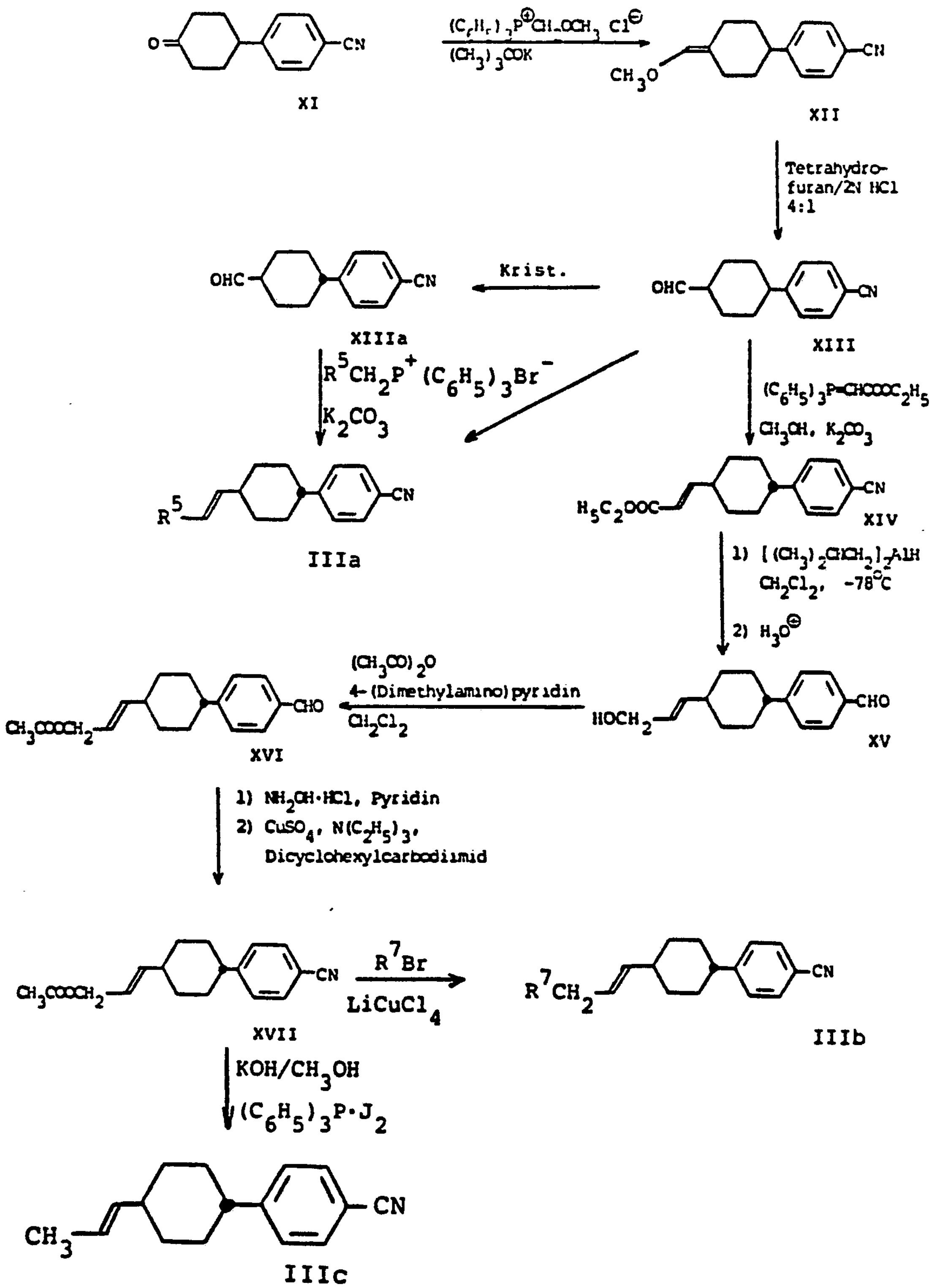

**Schema 3**

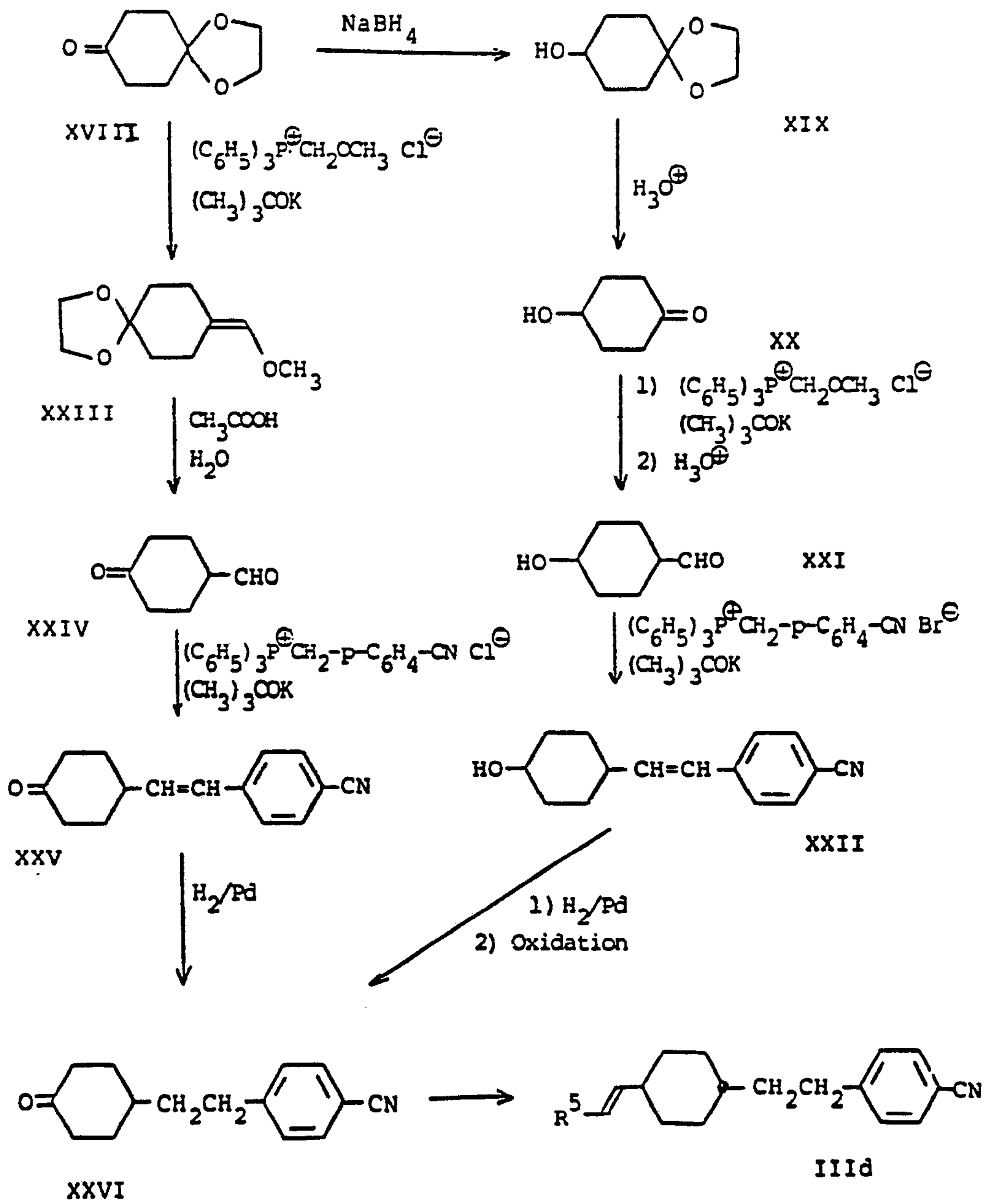
NaBH$_4$
XVIII
XIX
$(C_6H_5)_3P^{\oplus}CH_2OCH_3$ $Cl^{\ominus}$
$(CH_3)_3COK$
$H_3O^{\oplus}$
XX
XXIII
$CH_3COOH$
$H_2O$
1) $(C_6H_5)_3P^{\oplus}CH_2OCH_3$ $Cl^{\ominus}$
$(CH_3)_3COK$
2) $H_3O^{\oplus}$
XXIV
XXI
$(C_6H_5)_3P^{\oplus}CH_2$-p-$C_6H_4$-CN $Cl^{\ominus}$
$(CH_3)_3COK$
$(C_6H_5)_3P^{\oplus}CH_2$-p-$C_6H_4$-CN $Br^{\ominus}$
$(CH_3)_3COK$
XXV
XXII
$H_2$/Pd
1) $H_2$/Pd
2) Oxidation
XXVI
IIId

**Schema 4**

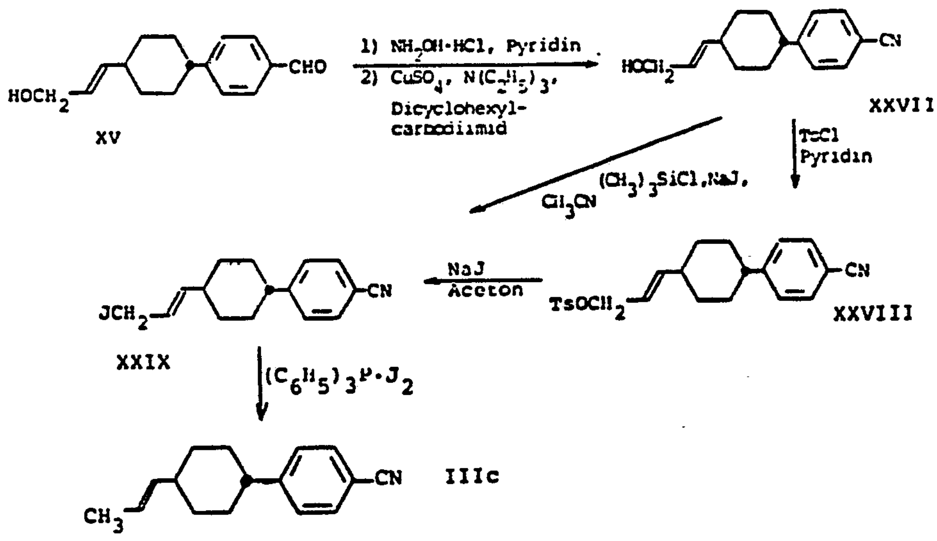


XV XXVII XXVIII XXIX IIIc

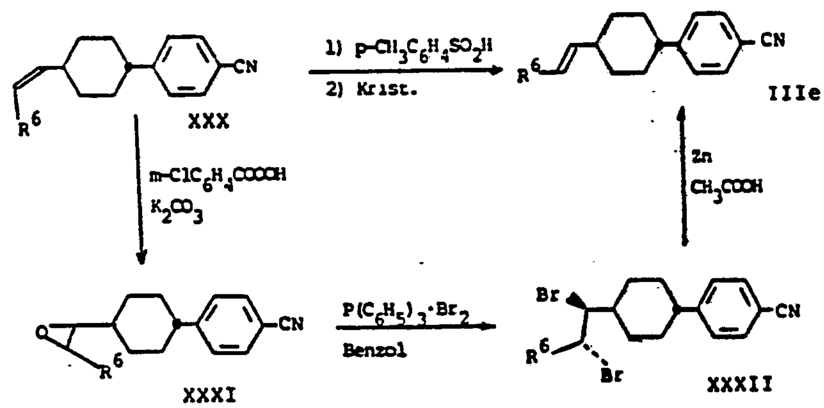


XXX IIIe XXXI XXXII

**Schema 5**

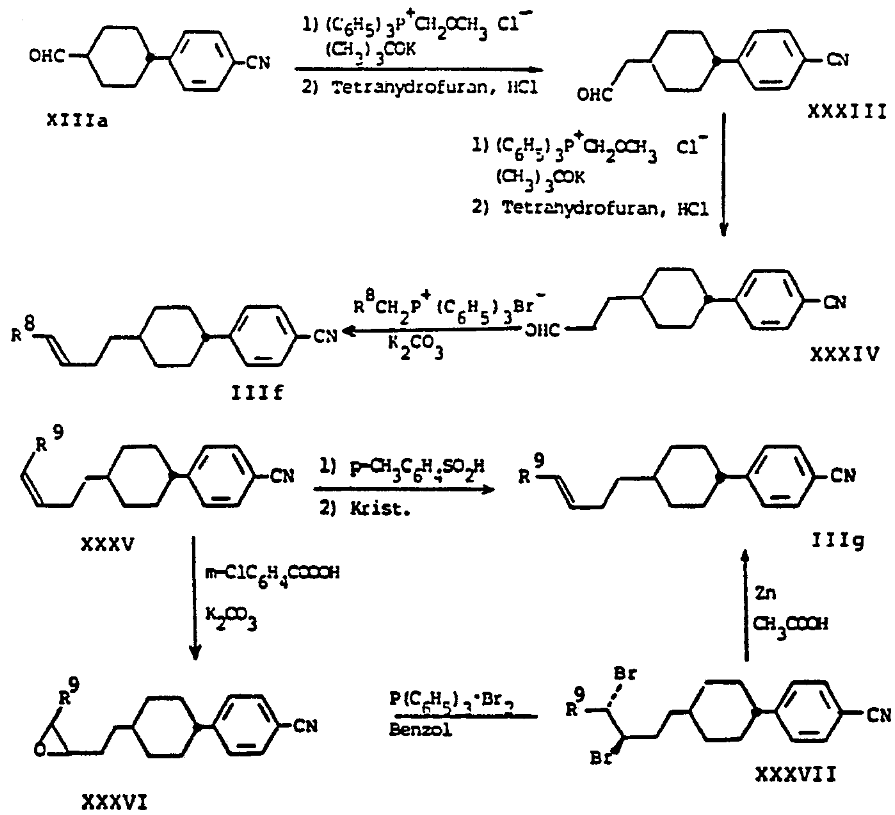


XIIIa, XXXIII, XXXIV, IIIf, XXXV, IIIg, XXXVI, XXXVII

**Schema 6**

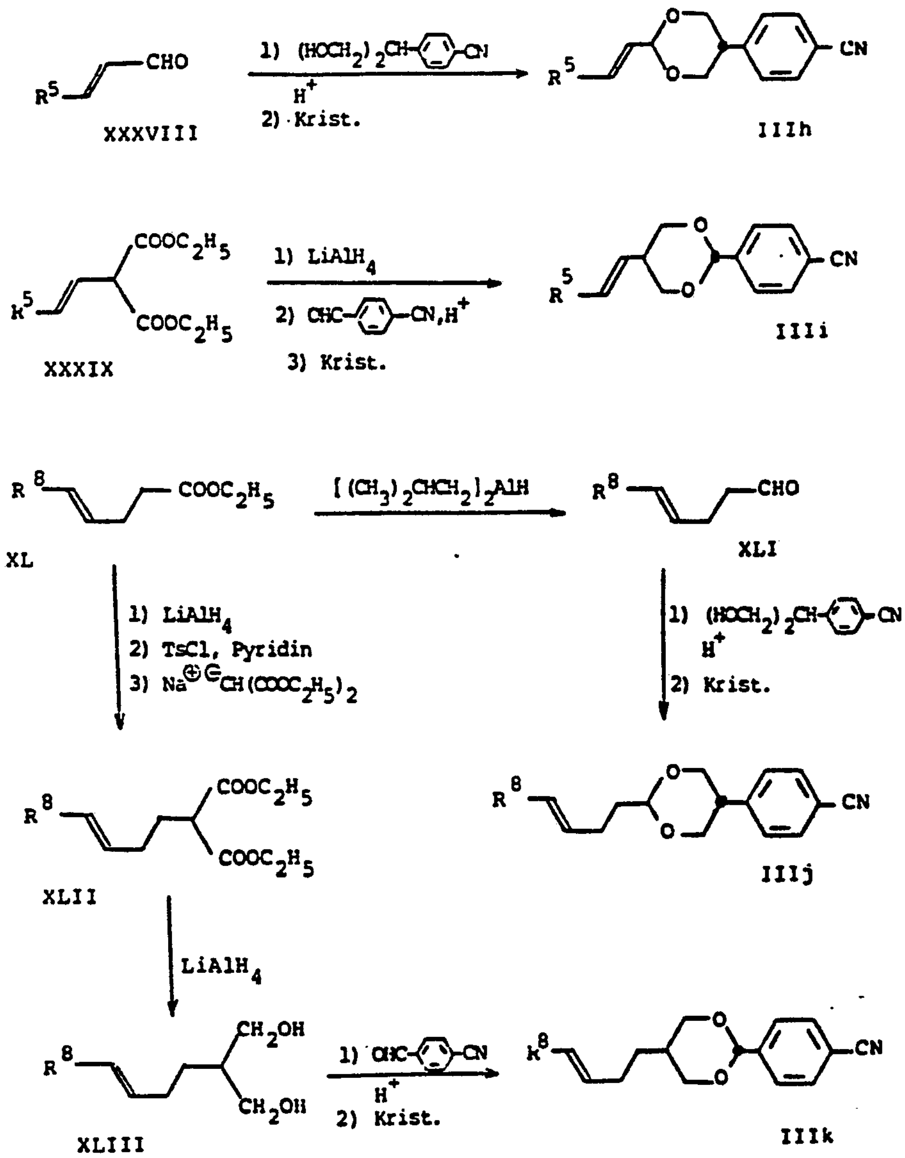
1) (HOCH2)2CH-⟨⟩-CN
H+
2) Krist.
XXXVIII
IIIh
1) LiAlH4
2) OHC-⟨⟩-CN,H+
3) Krist.
XXXIX
IIIi
[(CH3)2CHCH2]2AlH
XL
XLI
1) LiAlH4
2) TsCl, Pyridin
3) Na⊕⊖CH(COOC2H5)2
1) (HOCH2)2CH-⟨⟩-CN
H+
2) Krist.
XLII
IIIj
LiAlH4
1) OHC-⟨⟩-CN
H+
2) Krist.
XLIII
IIIk

**Schema 7**

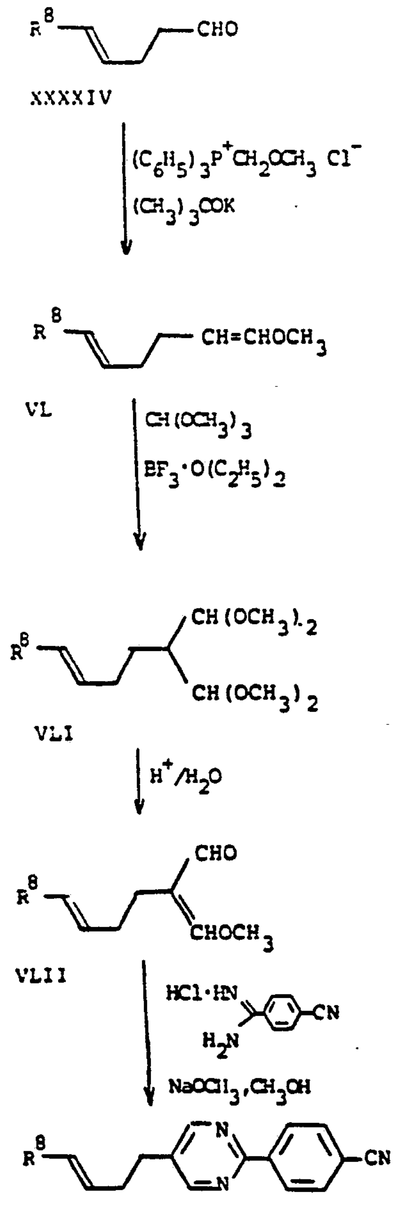


IIIl

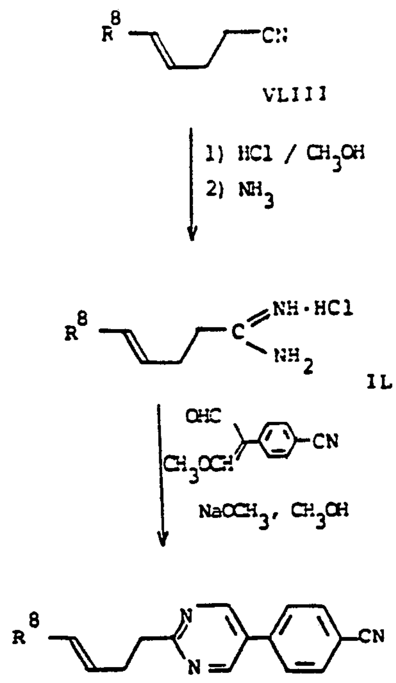


IIIm

Die Einführung anderer Abgangsgruppen anstelle der Acetoxygruppe in Formel XVII (oder analoger Verbindungen) kann nach an sich bekannten Methoden erfolgen. Beispiele für Jodid und Tosylat sind in Schema 4 angegeben. Gewünschtenfalls können derartige Verbindungen reduziert werden, z. B. nach den in Chem. Ber. 109, 1586 (1976) beschriebenen Methoden.

Bei der Herstellung der Verbindungen der Formeln IIIa und IIIf, worin $R^5$ und $R^8$ geradkettiges Alkyl bedeuten, gemäss Schema 2 bzw. 5 entsteht im allgemeinen ein Gemisch bestehend aus cis-Alkenyl- und trans-Alkenyl-Verbindung. Derartige Gemische können auf mit Silbernitrat beschichtetem Kieselgel chromatographisch getrennt werden. Gswünschtenfalls können die cis-Alkenyl-Verbindungen (oder Gemische mit überwiegend cis-Alkenyl-Verbindung) gemäss Schema 4 bzw. 5 in die entsprechenden trans-Alkenyl-Verbindungen übergeführt werden.

Die Umsetzung der Verbindung der Formel XXVI zu Verbindungen der Formel IIId gemäss Schema 3 kann in analoger Weise zu Schema 1 erfolgen.

Verbindungen der Formel III, welche eine Alkenylgruppe und einen Pyrimidinring aufweisen, können nach der in Schema 7 dargestellten Methode oder in analoger Weise zu den Schemata 2, 4 und 5 hergestellt werden.

Die Verbindungen der Formel I können in Form von Gemischen untereinander und/oder mit anderen Flüssigkristallkomponenten verwendet werden, wie z. B. mit Substanzen aus den Klassen der Schiffschen Basen, Azobenzols, Azoxybenzole, Phenylbenzoate, Cyclohexancarbonsäurephenylester, Cyclohexancarbonsäurecyclohexylester, Biphenyle, Terphenyle, Phenylcyclohexane, Phenylpyrimidine, Diphenylpyrimidine, Cyclohexylphenylpyrimidine, Phenyldioxane, 2-Cyclo-hexyl-1-phenyläthane und dergleichen. Derartige Substanzen sind dem Fachmann bekannt und viele davon sind zudem im Handel erhältlich. Insbesondere können die Verbindungen der Formel I auch im Gemisch mit Verbindungen der obigen Formel III verwendet werden.

Aufgrund der günstigen Eigenschaften der Verbindungen der Formel I und ihrer guten Mischbarkeit mit anderen Flüssigkristallkomponenten kann der Anteil der Verbindungen der Formel I in den erfindungsgemässen Mischungen in breiten Grenzen schwanken. Vorzugsweise enthalten die erfindungsgemässen Mischungen etwa 1-60 Gew.-% und besonders bevorzugt etwa 5-40 Gew.-% an Verbindungen der Formel I.

Bevorzugte Komponenten. welche im Gemisch mit einer oder mehreren Verbindungen der Formel I verwendet werden können, sind die Verbindungen der allgemeinen Formeln

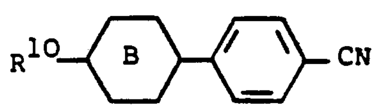
L

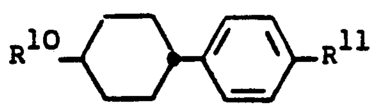
LI

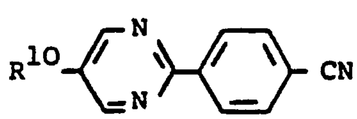
LII

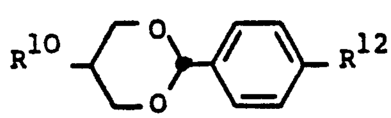
LIII

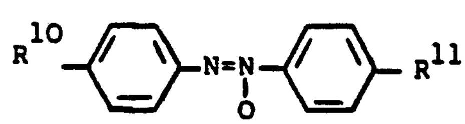
LIV

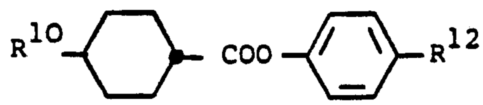
LV

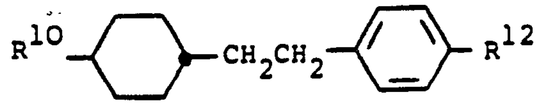

LVI

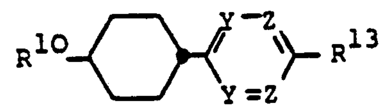

LVII

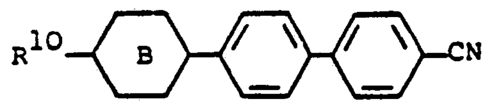

LVIII

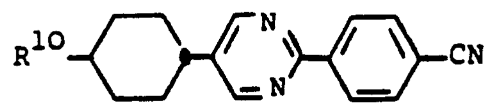

LIX

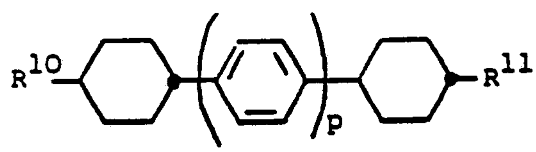

LX

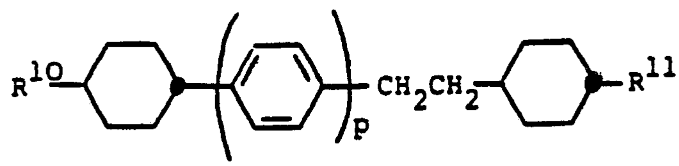

LXI

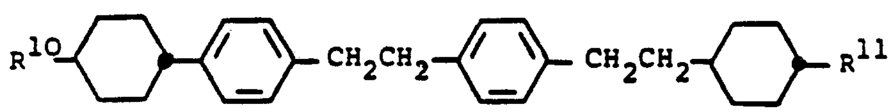

LXII

worin Ring B 1,4-Phenylen oder trans-1,4-Cyclohexylen darstellt, eines der Symbole Y und Z für N und das andere für CH steht, $R^{10}$ und $R^{11}$ geradkettiges $C_1$-$C_7$-Alkyl bezeichnen, $R^{12}$ Cyano oder geradkettiges $C_1$-$C_6$-Alkoxy bedeutet, $R^{13}$ geradkettiges $C_1$-$C_7$-Alkyl, p-($C_1$-$C_7$-Alkyl)phenyl oder trans-4-($C_1$-$C_7$-Alkyl)cyclohexyl bezeichnet und p für 0 oder 1 steht.

und die Verbindungen der obigen Formeln IIIa, IIIf, IIIi; IIIk und IIIl (Schemata 2 und 5-7).

Die erfindungsgemässen Mischungen können ferner geeignete, optisch aktive Verbindungen, beispielsweise optisch aktive Biphenyle, und/oder dichroitische Farbstoffe, beispielsweise Azo-. Azoxy- oder Anthrachinon-Farbstoffe, enthalten. Der Anteil solcher Verbindungen wird durch die Löslichkeit und die gewünschte Ganghöhe (pitch), Farbe, Extinktion und dergleichen bestimmt. Vorzugsweise beträgt der Anteil optisch aktiver Verbindungen höchstens etwa 4 Gew.-% und der Anteil dichroitischer Farbstoffe höchstens etwa 10 Gew.-%.

Die Herstellung der erfindungsgemässen flüssigkristallinen Gemische kann in an sich bekannter Weise erfolgen, z. B. durch Erhitzen einer Mischung der Bestandteile auf eine Temperatur knapp oberhalb des Klärpunktes und anschliessendes Abkühlen.

Die Herstellung elektro-optischer Vorrichtungen, welche als Dielektrikum eine erfindungsgemässe Mischung enthalten, kann ebenfalls in an sich bekannter Weise erfolgen, z. B. durch Evakuieren einer geeigneten Zelle und Einbringen der Mischung in die evakuierte Zelle.

Beispiele bevorzugter erfindungsgemässer Gemische sind die folgenden Mischungen. $V_{10}$ und $V_{50}$ bezeichnen die Spannung für 10 % bzw. 50 % Transmission, $p_o = (V_{50}-V_{10})/V_{10}$ ist ein Mass für die Steilheit der Transmissionskurve, $t_{on}$ und $t_{off}$ bezeichnet die Einschaltzeit bzw. die Ausschaltzeit (in einer Drehzelle bei 2,5 $V_{10}$ und Kippwinkel 0°) und $k_{11}$ (splay) und $k_{33}$ (bend) sind elastische Konstanten. Sofern nicht anders angegeben wurden die Messungen bei 22° C durchgeführt.

**Mischung 1**

| | | |
|---|---|---|
| 11,2 | Gew.-% | p-(5-Butyl-2-pyrimidinyl)benzonitril, |
| 3,4 | " | p-(5-Pentyl-2-pyrimidinyl)benzonitril, |
| 6,6 | " | p-(5-Heptyl-2-pyrimidinyl)benzonitril, |
| 13,35 | " | trans-4-Butylcyclohsxancarbonsäure-p-äthoxyphenylester, |
| 17,55 | " | trans-4-Butylcyclohexancarbonsäure-p-pentyloxyphenylester, |
| 12,3 | " | trans-4-Pentylcyclohexancarbonsäure-p-methoxyphenylester, |
| 19,05 | " | trans-4-Pentylcyclohexancarbonsäure-p-propyloxyphenylester, |
| 7,85 | " | p-[5-(trans-4-Äthylcyclohexyl)-2-pyrimidinyl]benzonitril, |
| 8,7 | " | p-[trans-4-(trans-1-Propenyl)cyclohexyl]phenylisothiocyanat; |

Smp. < -25° C, Klp. 62° C, nematisch; $V_{10}$=1,521 V, $p_o$=0,121; $k_{33}/k_{11}$=0,98.

**Mischung 2**

| | | |
|---|---|---|
| 6,5 | Gew.-% | p-(trans-4-Propylcyclohexyl)benzonitril, |
| 12,3 | " | p-[trans-4-(3-Butenyl)cyclohexyl]benzonitril, |
| 7,3 | " | p-[trans-4-(trans-3-Pentenyl)cyclohexyl]benzonitril, |
| 6,0 | " | 1-Äthyl-4-(trans-4-propylcyclohexyl)benzol, |
| 18,0 | " | 1-Äthoxy-4-[2-(trans-4-propylcyclohexyl)äthyl]benzol, |
| 4,7 | " | 4-Cyano-4'-(trans-4-pentylcyclohexyl)biphenyl, |
| 3,6 | " | 4-Cyano-4'-[trans-4-(3-butenyl)cyclohexyl]biphenyl, |
| 14,7 | " | 1-[2-(trans-4-Butylcyclohexyl)äthyl]-4-(trans-4-pentylcyclohexyl)benzol, |
| 5,0 | " | 4-[2-(trans-4-Butylcyclohexyl)äthyl]-4'-(trans-4-pentylcyclohexyl)biphenyl |
| 8,0 | " | 4-[2-(trans-4-Butylcyclohexyl)äthyl)-4'-(trans-4-pentylcyclohexyl)-1,1'-äthylendibenzol. |
| 13,9 | " | p-[trans-4-(trans-3-Pentenyl)cyclohexyl)-phenylisothiocyanat: |

Smp. < -25° C, Klp. 85° C. nematisch; $V_{10}$=2,51 V, $p_o$=0,127; $t_{on}$=31 ms, t=36 ms.

**Mischung 3**

| | | |
|---|---|---|
| 13,50 | Gew.-% | p-[trans-4-(trans-1-Butenyl)cyclohexyl]benzonitril, |
| 9,87 | " | p-[trans-4-(3-Butenyl)cyclohexyl]benzonitril, |
| 7,00 | " | 1-Äthyl-4-(trans-4-propylcyclohexyl)benzol, |
| 8,00 | " | 1-Äthoxy-4-(trans-4-propylcyclohexyl)benzol, |
| 5,00 | " | 1-Äthyl-4-[2-(trans-4-propylcyclohexyl)äthyl]benzol, |
| 3,59 | " | 4-Cyano-4'-(trans-4-pentylcyclohexyl)biphenyl, |
| 2,74 | " | 4-Cyano-4'-[trans-4-(3-butenyl)cyclohexyl]biphenyl, |
| 7,00 | " | 4-Propyl-4'-[trans-4-(trans-3-pentenyl)cyclohexyl]biphenyl, |
| 8,17 | " | p-[trans-4-(trans-3-pentenyl)cyclohexyl]benzoesäure-p'-propylphenylester, |
| 11,19 | " | 1-[2-(trans-4-Butylcyclohexyl)äthyl]-4-(trans-4-pentylcyclohexyl)benzol, |
| 5,00 | " | 4-[2-(trans-4-Butylcyclohexyl)äthyl]-4'-(trans-4-pentylcyclohexyl)biphenyl, |
| 6,00 | " | 4-[2-(trans-4-Butylcyclohexyl)äthyl]-4'-(trans-4-pentylcyclohexyl)-1,1'-äthylendibenzol, |
| 12,94 | " | p-[trans-4-(trans-3-Hexenyl)cyclohexyl]phenylisothiocyanat: |

Smp. < -30° C, Klp. 92° C, nematisch; $V_{10}$=2,32 V, $p_o$= 0,125; $t_{on}$=30 ms, $t_{off}$=40 ms.

**Mischung 4**

| | | |
|---|---|---|
| 13 | Gew.-% | p-(5-Butyl-2-pyrimidinyl)benzonitril, |
| 4 | " | p-(5-Pentyl-2-pyrimidinyl)benzonitril, |
| 7 | " | p-(5-Heptyl-2-pyrimidinyl)benzonitril, |
| 11 | " | trans-4-Butylcyclohexancarbonsäure-p-äthoxyphenylester, |
| 12 | " | trans-4-Butylcyclohexancarbonsäure-p-pentyloxyphenylester, |
| 10 | " | trans-4-Pentylcyclohexancarbonsäure-p-methoxyphenylester, |
| 16 | " | trans-4-Pentylcyclohexancarbonsäure-p-propyloxyphenylester, |
| 7 | " | p-[5-(trans-4-Äthylcyclohexyl)-2-pyrimidinyl]benzonitril, |
| 20 | " | p-(5-Pentyl-2-pyrimidinyl)phenylisothiocyanat; |

Smp. < -20°C, Klp. 59°C, nematisch; $V_{10}$=1,474 V, $p_o$=0,115.

**Mischung 5**

| | | |
|---|---|---|
| 10,27 | Gew.-% | p-(5-Butyl-2-pyrimidinyl)benzonitril, |
| 3,16 | " | p-(5-Pentyl-2-pyrimidinyl)benzonitril, |
| 5,53 | " | p-(5-Heptyl-2-pyrimidinyl)benzonitril, |
| 8,69 | " | trans-4-Butylcyclohexancarbonsäure-p-äthoxyphenylester, |
| 9,48 | " | trans-4-Butylcyclohexancarbonsäure-p-pentyloxyphenylester, |
| 7,90 | " | trans-4-Pentylcyclohexancarbonsäure-p-methoxyphenylester, |
| 12,64 | " | trans-4-Pentylcyclohexancarbonsäure-p-propyloxyphenylester, |
| 10,00 | " | 1-Äthyl-4-[2-(trans-4-propylcyclohexyl)äthyl]benzol, |
| 7,00 | " | 5-(trans-4-Äthylcyclohexyl)-2-(p-pentylphenyl)-pyrimidin, |
| 4,00 | " | 5-(p-Butylphenyl)-2-(p-pentylphenyl)pyrimidin, |
| 5,53 | " | p-[5-(trans-4-Äthylcyclohexyl)-2-pyrimidinyl]benzonitril, |
| 15,80 | " | p-(5-Pentyl-2-pyrimidinyl)phenylisothiocyanat: |

Smp. < -20°C, Klp. 59°C, nematisch; $V_{10}$=1,692 V, $p_o$=0,114; $k_{33}/k_{11}$=0,78.

Die Herstellung der Verbindungen der Formel I und der neuen Verbindungen der Formel III wird durch die folgenden Beispiele weiter veranschaulicht. C bedeutet eine kristalline, S eine smektische, $S_B$ eine smektisch B, N eine nematische und I die isotrope Phase.

**Beispiel 1**

6,77 g Triäthylammonium-p-(5-butyl-2-pyrimidinyl)-phenyldithiocarbamat wurden in 60 ml Chloroform und 2,5 ml Triäthylamin weitgehend gelöst. Anschliessend wurde das Gemisch unter Rühren und Kühlung mit einem Eisbad innert 5 Minuten tropfenweise mit 2,00 ml Chlorameisensäuremethylester versetzt. Nach 10 Minuten wurde das Eisbad entfernt. Nach weiteren 60 Minuten wurde dis gelbliche Lösung einmal mit 20 ml 0,5 N Salzsäure und dreimal mit je 20 ml Wasser gewaschen, über Natriumsulfat getrocknet und eingeengt. Chromatographie des Rückstandes an 60 g Kiesslgel mit Hexan/Äthylacetat (Vol. 95 : 5) ergab 3,65 g p-(5-Butyl-2-pyrimidinyl)phenylisothiocyanat, welches zur weiteren Reinigung zweimal aus Hexan umkristallisiert wurde. Ausbeute 3,17 g; Smp. (C-S) 60,8°C, Klp. (S-I) 77,8°C.

Das als Ausgangsmaterial verwendete Triäthylammonium-p-(5-butyl-2-pyrimidinyl)phenyldithiocarbamat wurde wie folgt hergestellt:

a) Eine Suspension von 12,62 g p-(5-Butyl-2-pyrimidinyl)benzoesäure in 150 ml Aceton wurde unter Kühlung mit einem Eis/Kochsalz-Bad (Innentemperatur -4°C) tropfenweise mit einer Lösung von 8,1 ml Triäthylemin in 25 ml Aceton und danach innert 15 Minuten tropfenweise mit einer Lösung von 4,9 ml Chlorameisensäuremethylester in 25 ml Aceton versetzt. Die Suspension wurde noch 75 Minuten gerührt und dann innert 10 Minuten tropfenweise mit einer Lösung von 4,90 g Natriumazid in 17,5 ml Wasser versetzt. Das Gemisch wurde noch 1 Stunde unter Kühlung mit dem Eis/Kochsalz-Bad und dann 30 Minuten ohne Kühlung gerührt. Das Gemisch wurde auf 300 ml Eiswasser gegossen und einmal mit 250 ml Diäthyläther und zweimal mit je 100 ml Diäthyläther extrahiert. Die Extrakte wurden mit 100 ml Wasser gewaschen, getrocknet und im Vakuum bei einer Badtemperatur von 35°C eingeengt, wobei 13,32 g festes, gelbliches p-(5-Butyl-2-pyrimidinyl)benzoesäureazid erhalten wurden. Das Azid wurde in 100 ml Toluol gelöst und die Lösung langsam in einen auf 90°C erhitzten Kolben getropft, wobei starke Stickstoffentwicklung einsetzte. Die Lösung wurde noch 1,5 Stunden auf 108°C erhitzt und dann eingeengt. Es resultierten 11,59 g rohes, hellgelbes p-(5-Butyl-2-pyrimidinyl)phenylisocyanat; Smp. 48°C.

b) 8,11 g rohes p-(5-Butyl-2-pyrimidinyl)phenylisocyanat wurden in kleinen Portionen in 40 ml auf 60°C erwärmte 8N Salzsäure eingetragen, wobei starkes Schäumen eintrat. Nach beendeter Zugabe wurde das gemisch 4 Stunden zu leichtem Sieden erhitzt und dann auf Eis und einen Überschuss von festem

Natriumcarbonat gegossen. Die wässrige Phase wurde einmal mit 300 ml Diäthyläther und zweimal mit je 100 ml Diäthyläther extrahiert. Die vereinigten Extrakte wurden filtriert, Das Filtrat wurde zweimal mit Wasser gewaschen, über Natriumsulfat getrocknet und eingeengt, wobei 5,16 g gelbliches, kristallines p-(5-Butyl-2-pyrimidinyl)anilin erhalten wurden; Smp. 154,5-155,5°C.

c) Eine Lösung von 5,10 g p-(5-Butyl-2-pyrimidinyl)anilin in 30 ml Dioxan, 1,70 ml Schwefelkohlenstoff und 3,44 ml Triäthylamin wurde 90 Stunden in den Kühlschrank gestellt (0°C). Der erhaltene, gelbe Niederschlag wurde abfiltriert, mit Diäthyläther gewaschen und getrocknet. Es resultierten 6,77 g Triäthylammonium-p-(5-butyl-2-pyrimidinyl)phenyldithiocarbamat, welches ohne zusätzliche Reinigung weiterverwendet wurde.

In analoger Weise wurden folgende Verbindungen hergestellt:

p-(5-Pentyl-2-pyrimidinyl)phenylisothiocyanat; Smp. (C-S) 46°C, Klp. (S-I) 85,5°C.

p-[5-(trans-4-Äthylcyclohexyl)-2-pyrimidinyl-phenylisothiocyanat; Smp. (C-S) 114,0°C, Phasenübergang S-N 159,5°C. Klp. (N-I) 228,5°C.

p-(2-Pentyl-5-pyrimidinyl)phenylisothiocyanat; Smp. (C-I) 92°C. Klp. (N-I) 43,5°C.

**Beispiel 2**

428 mg Triäthylammonium-p-[trans-4-(trans-1-pentenyl)cyclohexyl]phenyldithiocarbamat wurden in 4 ml Chloroform gelöst und unter Argon mit 284 µl Triäthylamin versetzt. Anschliessend wurde das Gemisch auf 0°C gekühlt und tropfenweise mit 194 µl Chlorameisensäureäthylester versetzt. Das Gemisch wurde noch 10 Minuten bei 0°C und dann 1 Stunde bei Raumtemperatur gerührt. Anschliessend wurde das Reaktionsgemisch zwischen 100 ml Diäthyläther und 100 ml Wasser verteilt. Die wässrige Phase wurde zweimal mit je 100 ml Diäthyläther nachextrahiert. Die organischen Phasen wurden zweimal mit je 100 ml Wasser gewaschen, über Magnesiumsulfat getrocknet und eingeengt. Wiederdruckchromatographie (0,5 bar) des öligen Rückstandes (450 mg) an 35 g Kieselgel mit Äthylacetat/Petroläther (Vol. 7 : 93) ergab 234 mg (51 %) p-[trans-4-(trans-1-Pentenyl)cyclohexyl]phenylisothiocyanat als farblose Kristalle. Durch Umkristallisation aus 10 ml Methanol bei 0°C wurden 119 mg p-[trans-4-(trans-1-Pentenyl)cyclohexyl]phenylisothiocyanat mit Smp. (C-I) 61,2°C und Klp. (W-I) 49,0°C erhalten.

Das als Ausgangsmaterial verwendete Triäthylammonium-p-[trans-4-(trans-1-pentenyl)cyclohexyl]phenyldithiocarbamat wurde wie folgt hergestellt:

a) In einem Rundkolben mit Magnetrührer und Rückflusskühler wurde unter Argonbegasung ein Gemisch von 100 mg p-[trans-4-(trans-1-Pentenyl)cyclohexyl]benzonitril und 10 ml einer 10 %-igen Lösung von Kaliumhydroxid in Diäthylenglykol 1 Stunde bei 180°C gekocht. Nach Abkühlen wurde das braune Reaktionsgemisch mit 25 %-iger Salzsäure sauer gestellt und zwischen 70 ml Dichlormethan und 70 ml Wasser verteilt. Die wässrige Phase wurds dreimal mit je 70 ml Dichlormethan nachextrahiert. Die organischen Phasen wurden zweimal mit je 70 ml Wasser gewaschen und über Magnesiumsulfat und Aktivkohle getrocknet. Nach Abtrennung des Lösungsmittels im Rotationsverdampfer wurden 99 mg (92 %) p-[trans-4-(trans-1-Pentenyl)cyclohexyl]benzoesäure als weisse, intensiv riechende Kristalle erhalten; Rf-Werte 0,44 (Äthylacetat/Petroläther Vol. 3 : 7).

b) Eine Suspension von 558 mg p-[trans-4-(trans-1-Pentenyl)cyclohexyl]benzoesäure in 20 ml Aceton und 1 ml Wasser wurde auf 0°C gekühlt und langsam mit einer Lösung von 571 µl Triäthylamin in 4 ml Aceton versetzt, wobei sich eine klare Lösung bildete. Anschliessend wurde das Gemisch tropfenweise mit einer Lösung von 487,9 µl Chlorameisensäureäthylester in 2 ml Aceton versetzt, wobei ein weisser flockiger Niederschlag ausfiel. Das Gemisch wurde noch 30 Minuten bei 0°C gerührt, dann tropfenweise mit einer Lösung von 366,2 mg Natriumazid in 2 ml Wasser versetzt und noch 1 Stunde bei 0°C weitergerührt. Danach wurde das Reaktionsgemisch zwischen 150 ml Diäthyläther und 150 ml Wasser verteilt. Die wässrige Phase wurde zweimal mit je 150 ml Diäthyläther nachextrahiert. Die organischen Phasen wurden zweimal mit je 100 ml Wasser gewaschen, über Magnesiumsulfat getrocknet und eingeengt. Wiederdruckchromatographie (0,5 bar) des grünlichen, flüssigkristallinen Rückstandes (590 mg) an 70 g Kiesslgel mit Äthylacetat/Petroläther (Vol. 1 : 9) ergab 539 mg (89 %) p-[trans-4-(trans-1-Pentenyl)cyclohexyl]benzoesäureazid als farblose Kristalle; Rf-Wert 0,53 (Äthylacetat/Petroläther Vol. 1 : 9).

c) Ein Gemisch von 539 mg p-[trans-4-(trans-1-Pentenyl)cyclohexyl]benzoesäureazid und 10 ml Propanol wurde unter Argonbegasung 2,5 Stunden am Rückfluss gekocht und danach eingeengt. Wiederdruckchromatographie (0,5 bar) des weissen, kristallinen Rückstandes an 70 g Kieselgel mit Äthylacetat/Petroläther (Vol. 1 : 9) ergab 515 mg (86 %) Propyl-p-[trans-4-(trans-1-Pentenyl)cyclohexyl]phenylcarbamat als farblose Kristalle; Rf-Wert 0,33 (Äthylacetat/Petroläther Vol. 1 : 9).

d) Ein Gemisch von 118 mg Propyl-p-[trans-4-(trans-1-Pentenyl)cyclohexyl]phenylcarbamat und 12,5 ml einer 10 %-igen Lösung von Kaliumhydroxid in Diäthylenglykol/Wasser (Vol. 4 : 1) wurde 1 Stunde bei 120°C gekocht, dann auf Raumtemperatur abgekühlt und zwischen 70 ml Diäthyläther und 70 ml Wasser verteilt. Die wässrige Phase wurde zweimal mit je 70 ml Diäthyläther nachextrahiert. Die organischen Phasen wurden zweimal mit je 50 ml Wasser gewaschen, über Magnesiumsulfat getrocknet und eingeengt. Wiederdruckchromatographie (0,5 bar) des braunen, kristallinen Rückstandes (150 mg) an 35 g Kieselgel mit Äthylacetat/Petroläther (Vol. 15 : 85) ergab 76,6 mg (88 %) -[trans-4-(trans-1-Pentenyl)cyclohexyl]anilin als leicht gelbliche Kristalle; Reinheit 97 %; Rf-Wert 0,24 (Äthylacetat/Petroläther Vol. 1 : 5).

e) Eine Lösung von 390 mg p-[trans-4-(trans-1-Pentenyl)-cyclohexyl]anilin (Reinheit 93 %) in 4 ml Hexan wurde der Reihe nach mit 446 µl Triäthylamin und 214 µl Schwefelkohlenstoff versetzt und dann 65 Stunden bei 0°C stehengelassen. Der erhaltene gelbe Wiederschlag wurde abfiltriert, zweimal mit Hexan gewaschen und am Hochvakuum getrocknet. Es resultierten 428 mg Triäthylammonium-p-[trans-4-(trans-1-pentenyl)cyclohexyl]phenyldithiocarbamat, welches ohne zusätzliche Reinigung direkt weiterverwendet wurde.

In analoger Weise wurden folgende Verbindungen hergestellt:
p-[trans-4-(trans-1-Propenyl)cyclohexyl]phenylisothiocyanat; Smp. (C-I) 88,5°C, virtueller Klp. 47°C,
p-[trans-4-(trans-3-Pentenyl)cyclohexyl]phenylisothiocyanat; Smp. (C-N) 59,2°C, Klp. (N-I) 65,2°C,
p-[trans-4-(trans-1-Butenyl)cyclohexyl]phenylisothiocyanat; Smp. (C-I) 48.2°C. Klp. (N-I) 35,6°C,
p-[trans-4-(trans-1-Hexenyl)cyclohexyl]phenylisothiocyanat; Smp. (C-N) 16,3°C, Klp. (N-I) 23,5°C,
p-[trans-4-(3-Butenyl)cyclohexyl]phenylisothiocyanat; Smp. (C-N) 24,6°C, Klp. (N-I) 43,7°C,
p-[trans-4-(trans-3-Hexenyl)cyclohexyl]phenylisothiocyanat; Smp. (C-N) 24,4°C, Klp. (N-I) 37,2°C,
4'-(3-Butenyl)-4-biphenylylisothiocyanat;
4'-(trans-3-Pentenyl)-4-biphenylylisothiocyanat;
p-[2-(trans-4-Propylcyclohexyl)äthyl]phenylisothiocyanat; Smp. (C-I) 43,0°C, Klp. (N-I) 38,6°C,
p-[2-(trans-4-Butylcyclohexyl)äthyl]phenylisothiocyanat; Smp. (C-N) 23,5°C, Klp. (N-I) 33,6°C,
p-[2-(trans-4-Pentylcyclohexyl)äthyl]phenylisothiocyanat; Smp. (C-N) 41,0°C, Klp. (N-I) 47,5°C,
p-[2-(trans-4-Hexylcyclohexyl)äthyl]phenylisothiocyanat; Smp. (C-I) 50,0°C, Klp. (N-I) 41,4°C,
p-[2-(trans-4-Heptylcyclohexyl)äthyl]phenylisothiocyanat: Smp. (C-I) 56,2°C, Klp. (N-I) 49,2°C,
4'-(trans-4-Aethylcyclohexyl)-4-biphenylylisothiocyanat; Smp. (C-N) 151,0°C, Klp. (N-I) 214,5°C,
4'-(trans-4-Propylcyclohexyl)-4-biphenylylisothiocaynat: Smp. (C-N) 149,3°C, Klp. (N-I) 240,5°C,
4'-(trans-4-Butylcylohexyl)-4-biphenylylisothiocyanat; Smp. (C-S) 119,8°C, Phasenübergang S-N 132,5°C, Klp. (N-I) 233,0°C,
4'-(trans-4-Pentylcyclohexyl)-4-biphenylylisothiocyanat; Smp. (C-S) 123,0°C, Phasenübergang S-N 133,0°C, Klp. (N-I) 233,5°C,
4'-(trans-4-Hexylcyclohexyl)-4-biphenylylisothiocyanat; Smp. (C-S) 96,2°C, Phasenübergang S-N 147,0°C, Klp. (N-I) 223,0°C,
4'-(trans-4-Heptylcyclohexyl)-4-biphenylylisothiocyanat; Smp. (C-S) 108,8°C, Phasenübergang S-N 156,0°C, Klp. (N-I) 219,5°C.

**Beispiel 3**

In einem Sulfierkolben mit Thermometer, Tropftrichter und Serumkappe wurde unter Argonbegasung eine Lösung von 7,1 mMol Äthylmagnesiumbromid (hergestellt aus 172 mg Magnesium und 530 µl Äthylbromid) in 20 ml absolutem Tetrahydrofuran bei -78°C vorgelegt und der Reihe nach mit 3,6 ml einer 0,48M Lösung von Dilithiumtetrachlorokuprat in absolutem Tetrahydrofuran und mit einsr Lösung von 500 mg p-[trans-4-(3-Acetoxy-trans-1-propenyl)cyclohexyl)benzonitril in 10 ml absolutem Tetrahydrofuran versetzt. Nach beendeter Zugabe wurde das Reaktionsgemisch auf -15°C erwärmt, bei dieser Temperatur noch 1,5 Stunden gerührt, anschliessend mit 20 ml gesättigter Ammoniumchlorid-Lösung versetzt und noch 1 Stunde bei Raumtemperatur gerührt. Die nunmehr tiefblaue, wässrige Phase wurde abgetrennt und noch zweimal mit je 50 ml Diäthyläther extrahiert. Die organischen Phasen wurden zweimal mit je 50 ml Wasser gewaschen, über Magnesiumsulfat getrocknet und eingeengt. Wiederdruckchromatographie (0,5 bar) des Rückstandes (0,4 g) an Kieselgel mit Essigester/Petroläther (Vol. 3 : 97) ergab 375 mg rohes p-[trans-4-(trans-1-Pentenyl)cyclohexyl]benzonitril, welches gemäss gaschromatographischer Analyse zu 8,5 % mit p-[trans-4-(trans-1-Pentenyl)cyclohexyl]propiophenon und zu 4,0 % mit p-[trans-4-(1-Vinylpropyl)cyclohexyl]benzonitril verunreinigt war. Behandlung dieses Rohproduktes mit einem Überschuss Natriumborhydrid in Methanol bei 0°C (zwecks Reduktion des Propiophenons), Aufarbeitung, Niederdruckchromatographie (0,5 bar) an Kieselgel mit Essigester/Petroläther (Vol. 1 : 9) und schliesslich Umkristallisation aus Methanol bei -78°C ergab p-[trans-4-(trans-1-Pentenyl)cyclohexyl]benzonitril in 98,6 %-iger Reinheit; Smp. (C-N) 15,5°C, Klp. (N-I) 57,0°C.

Das als Ausgangsmaterial verwendete p-[trans-4-(3-Acetoxy-trans-1-propenyl)cyclohexyl]benzonitril wurde wie folgt hergestellt:

a) In einem Sulfierkolben mit Thermometer, mechanischem Rührer, Tropftrichter und Festsubstanzzugaberohr wurden unter Argonbegasung 10,4 g Triphenyl-methoxymethyl-phosphoniumchlorid in 60 ml t-Butylmethyläther aufgeschlämmt und bei -10°C innert 10 Minuten mit 3,6 g festem Kalium-t-butylat versetzt. Nach beendeter Zugabe wurde noch während 30 Minuten bei -10°C bis 0°C gerührt und dann das tieforange, heterogene Reaktionsgemisch bei 0°C tropfenweise mit einer Lösung von 4,2 g 4-(p-Cyanophenyl)cyclohexanon in 50 ml absoluten Tetrahydrofuran versetzt. Anschliessend wurde das Reaktionsgemisch noch 2 Stunden bei Raumtemperatur gerührt, dann auf 500 ml Hexan gegossen und filtriert. Niederdruckchromatographie (0,5 bar) des eingeengten Rückstandes (7,1 g) an Kieselgel mit Essigsster/Petroläther (Vol. 5 : 95) ergab 4,5 g (94 %) p-[4-(Methoxymethylen)cyclohexyl]benzonitril als farbloses Öl; Reinheit 95 %, Rf-Wert (Essigester/Petroläther Vol. 1 : 9) 0,30.

b) In einem Rundkolben wurde ein Gemisch von 4,2 g p-[4-(Methoxymethylen)cyclohexyl]benzonitril und 100

ml Tetrahydrofuran/2N Salzsäure (Vol. 4 : 1) während 30 Minuten zum Rückfluss erhitzt. Anschliessend wurde das Reaktionsgemisch auf 100 ml Wasser gegossen und dreimal mit je 100 ml Diäthyläther extrahiert. Die organischen Phasen wurden noch einmal mit 100 ml Wasser gewaschen, über Magnesiumsulfat getrocknet und eingeengt. Es resultierten 3,9 g (100 %) 4-(p-Cyanophenyl)cyclohexancarboxaldehyd als farbloses Öl, welches ohne weitere Reinigung in der nächsten Stufe eingesetzt wurde; trans/cis-Verhältnis ca. 3 : 1, Rf-Wert (Essigester/Petroläther Vol. 3 : 7) 0,41. Durch Kristallisation aus Hexan konnte reiner trans-4-(p-Cyanophenyl)cyclohexancarboxaldehyd erhalten werden; Smp. 57,1°C.

c) In einem Sulfierkolben mit Festsubstanzzugaberohr wurde unter Argonbegasung ein Gemisch von 3,9 g des oben erhaltenen 4-(p-Cyanophenyl)cyclohexancarboxaldehyds und 272 mg gemahlenem Kaliumcarbonat in 60 ml Äthanol bei Raumtemperatur vorgelegt und innert 15 Minuten mit 7,6 g festem Äthoxycarbonylmethylentriphenylphosphoran versetzt. Anschliessend wurde das Reaktionsgemisch noch 2 Stunden bei Raumtemperatur gerührt, dann am Rotationsverdampfer von Äthanol befreit, in 100 ml Wasser aufgenommen und dreimal mit je 100 ml Methylenchlorid extrahiert. Die organischen Phasen wurden noch zweimal mit je 100 ml Wasser gewaschen, über Magnesiumsulfat getrocknet und eingeengt. Wiederdruckchromatographie (0,5 bar) des Rückstandes (12 g) an Kieselgel mit Toluol/Petroläther/Essigester (Vol. 5 : 4 : 1) ergab 5,2 g einer kristallinen Masse, welche nach Kristallisation aus 500 ml Hexan 3,9 g (75 %) trans-3-[trans-4-(p-Cyanophenyl)cyclohexyl]acrylsäure-äthylester als farblose Kristalle mit Smp. 125°C lieferte.

d) In einem Sulfierkolben mit Thermometer und Serumkappe wurde unter Argonbegasung eine Lösung von 1,0 g trans-3-[trans-4-(p-Cyanophenyl)cyclohexyl]acrylsäure-äthylester in 25 ml Methylenchlorid bei -78°C vorgelegt und innert 10 Minuten mit 15,0 ml einer 0,84M Lösung von Diisobutylaluminiumhydrid in Toluol versetzt. Nach beendeter Zugabe wurde das Reaktionsgemisch auf -10°C erwärmt und noch 30 Minuten bei dieser Temperatur gerührt, bevor es auf 100 ml 0,2N Schwefelsäure gegossen und zweimal mit je 50 ml Methylenchlorid extrahiert wurde. Die organischen Phasen wurden mit 50 ml Wasser gewaschen, über Magnesiumsulfat getrocknet und eingeengt. Der Rückstand (ca. 850 mg) wurde in 30 ml Methylenchlorid gelöst und der Reihe nach mit 0,5 ml Essigsäureanhydrid und 45 mg 4-(Dimethylamino)pyridin versetzt. Das Reaktionsgemisch wurde 1 Stunde bei Raumtemperatur gerührt, dann auf 50 ml gesättigte, wässrige Kupfersulfatlösung gegossen und zweimal mit je 50 ml Methylenchlorid extrahiert. Die organischen Phasen wurden noch zweimal mit je 50 ml Wasser gewaschen, über Magnesiumsulfat getrocknet und eingeengt. Niederdruckchromatographie (0,5 bar) des Rückstandes (0,95 g) an Kieselgel mit Essigester/Petroläther (Vol. 1: 9) ergab 720 mg (71 %) p-[trans-4-(3-Acetoxy-trans-1-propenyl)cyclohexyl]benzaldehyd; Reinheit 99,9 %, Rf-Wert (Essigester/Petroläther Vol. 1 : 9) 0,31.

e) In einem Sulfierkolben mit mechanischem Rührer wurde unter Argonbegasung eine Lösung von 513 mg Hydroxylammoniumchlorid in 5 ml Wasser vorgelegt und bei Raumremperatur mit einer Lösung von 2,0 g p-[trans-4-(3-Acetoxy-trans-1-propenyl)cyclohexyl]benzaldehyd in 10 ml Pyridin versetzt. Das Reaktionsgemisch wurde 1 Stunde gerührt und dann der Reihe nach mit 350 mg Kupfersulfat-pentahydrat und einer Lösung von 2,1 ml Triäthylamin in 10 ml Methylenchlorid versetzt. Nachdem die zunächst tintenblaue Farbe des Kupfer-Pyridin-Komplexes nach olivgrün umgeschlagen war, wurde eine Lösung von 1,74 g Dicyclohexylcarbodiimid in 20 ml Methylenchlorid zugesetzt. Anschliessend wurde das Reaktionsgemisch noch während 3 Stunden bei Raumtemperatur gerührt und dann filtriert. Das Filtrat wurde auf 100 ml Wasser gegossen und dreimal mit je 100 ml Methylenchlorid extrahiert. Die organischen Phasen wurden noch zweimal mit je 50 ml Wasser gewaschen, über Magnesiumsulfat getrocknet und eingeengt. Wiederdruckchromatographis (0,5 bar) des Rückstandes (2,75 g) an Kieselgel mit Toluol/Essigester (Vol. 9 : 1) ergab 2,26 g (114 %) p-[trans-4-(3-Acetoxy-trans-1-propenyl)cyclohexyl]benzonitril als farblose Kristalle, welche als einige Verunreinigung noch etwas Dicyclohexylcarbodiimid enthielten. Dieses Material wurde ohne zusätzliche Reinigung weiterverwendet. Rf-Wert (Toluol/Essigester Vol. 9 : 1) 0,33.

In analoger Weise wurde folgende Verbindung hergestellt:

p-[trans-4-(trans-1-Butenyl)cyclohexyl]benzonitril, Smp. (C-N) 44,2°C, Klp. (N-I) 49,5°C.

**Beispiel 4**

In einem Rundkolben mit Rückflusskühler wurde unter Argonbegasung ein Gemisch von 3,8 g 4-(p-Cyanophenyl)cyclohexancarboxaldehyd (hergestellt nach Beispiel 3) 10,3 g Propyltriphenylphosphoniumbromid und 12,3 g Kaliumcarbonat in 200 ml Dioxan während 25 Stunden zum Rückfluss erhitzt. Anschliessend wurde das abgekühlte Reaktionsgemisch filtriert, eingeengt, in 150 ml Wasser aufgenommen und dreimal mit je 150 ml Diäthyläther extrahiert. Die organischen Phasen wurden noch zweimal mit je 100 ml Wasser gewaschen, über Magnesiumsulfat getrocknet und eingeengt. Wiederdruckchromatographie (0,5 bar) des Rückstandes (10,5 g) mit Essigester/Petroläther (Vol. 3 : 97) ergab 2,35 g (55 %) farblose, semikristalline Masse, welche gemäss gaschromatographischer Analyse aus 80,3 Gew.-% p-[trans-4-(cis-1-Butenyl)cyclohexyl]benzonitril, 17,5 Gew.-% p-[trans-4-(trans-1-Butenyl)cyclohexyl]benzonitril und 2,2 Gew.-% p-[cis-4-(cis-1-Butenyl)cyclohexyl]benzonitril bestand. Durch zusätzliche Niederdruckchromatographie (0,5 bar) dieses Materials an mit 10 % Silbernitrat beschichtetem Kieselgel unter Verwendung von Essigester/Petroläther (Vol. 3:97) sowie durch anschliessende Kristallisation aus Methanol bei -78°C konnte reines p-[trans-4-(trans-1-Butenyl)cyclohexyl]benzonitril isoliert werden: Smp

(C-N) 44 2°C, Klp. (N-I) 49,5°C.

**Beispiel 5**

In einem Sulfierkolben mit Tropftrichter und Thermometer wurde unter Argonbegasung eine Suspension von 2,51 g Methyltriphenylphosphoniumbromid in 80 ml absolutem Tetrahydrofuran bei -20°C vorgelegt und mit 7,6 ml einer ca. 0,8M Lösung von Butyllithium in Hexan versetzt. Nach 30 Minuten Rühren bei -20°C wurde zu dem gelben Reaktionsgemisch eine Lösung von 1,0 g trans-4-(p-Cyanophenyl)cyclo-hexancarboxaldehyd in 10 ml absolutem Tetrahydrofuran bei -20°C innert 5 Minuten zugetropft, wobei die gelbe Farbe verschwand. Nun wurde noch 30 Minuten bei -20°C nachgerührt und dann der Kolbeninhalt auf 100 ml Wasser gegossen und dreimal mit je 100 ml Diäthyläther extrahiert. Die organischen Phasen wurden zweimal mit je 100 ml Wasser gewaschen, über Magnesiumsulfat getrocknet und eingeengt. Niederdruckchromatographie (0,5 bar) des Rückstandes (2,3 g) an Kieselgel mit Essigester/Petroläther (Vol. 3 : 97) ergab 897 mg (91 %) p-(trans-4-Vinylcyclohexyl)benzonitril als farblose Kristalle; Reinheit 99,4 %. Durch zusätzliche Kristallisation aus 22 ml Methanol wurde p-(trans-4-Vinylcyclohexyl)-benzonitril in einer Reinheit von 99,95 % erhalten; Smp. (C-I) 56,4°C, Klp. 28,5°C.

**Beispiel 6**

In einem Sulfierkolben mit Thermometer, mechanischem Rührer, Tropftrichter und Festsubstanzzugaberohr wurde unter Argonbsgasung eine Suspension von 3,6 g Butyltriphenylphosphoniumbromid in 40 ml t-Butylmethyläther bei Raumtemperatur vorgelegt, mit 1,01 g Kalium-t-butylat versetzt und noch 1 Stunde bei Raumtemperatur gerührt. Anschliessend wurde das tieforange, heterogene Reaktionsgemisch auf -60°C abgekühlt und innert 15 Minuten mit einer Lösung von 1,28 g trans-4-(p-Cyanophenyl)cyclohexancarboxaldehyd in 10 ml t-Butylmethyläther versetzt. Das Reaktionsgemisch wurde noch 60 Minuten unter langsamem Erwärmen auf -30°C gerührt, dann auf 100 ml Wasser gegossen und dreimal mit je 50 ml Diäthyläther extrahiert. Die organischen Phasen wurden einmal mit 50 ml Wasser gewaschen, über Magnesiumsulfat getrocknet und eingeengt. Niederdruckchromatographie (0,5 bar) des Rückstandes (3,45 g) an Kieselgel mit Essigester/Petroläther (Vol. 3 : 97) ergab 1,52 g (99 %) p-[trans-4-(1-Pentenyl)cyclohexyl]benzonitril(trans-1-Pentenyl/cis-1-Pentenyl-Verhältnis ca. 5 : 95) als farbloses Öl; Rf-Wert (Essigester/Petroläther Vol. 3 : 97) 0,19.

**Beispiel 7**

In einem Rundkolben mit Magnetrührer und Rückflusskühler wurde unter Argonbegasung ein Gemisch von 3,79 g p-[trans-4-(1-Hexenyl)cyclohexyl]benzonitril (hergestellt in analoger Weise zu Beispiel 6; trans-1-Hexenyl/cis-1- Hexenyl-Verhältnis ca. 5 : 95) und 758 mg Benzolsulfinsäure in 50 ml 1,4-Dioxan 15 Stunden unter Rückfluss gekocht. Anschliessend wurde nochmals 379 mg Benzolsulfinsäure zugegeben und das Gemisch weitere 4 Stunden zum Rückfluss erhitzt. Dann wurde das abgekühlte Reaktionsgemisch auf 50 ml 1N Natronlauge gegossen und dreimal mit je 100 ml Hexan extrahiert. Die organischen Phasen wurden zweimal mit je 50 ml Wasser gewaschen, über Magnesiumsulfat getrocknet und singeengt. Dreimalige Kristallisation des quantitativ zurückerhaltenen, äquilibrierten Olefingemisches (trans-1-Hexenyl/cis-1-Hexenyl-Verhältnis 80,4 : 19,6) aus Methanol ergab schliesslich 1,74 g (46 %) p-[trans-4-(trans-1-Hexenyl)cyclohexyl]benzonitril (enthaltend 0,3 % cis-1-Hexenyl-Isomer) mit Smp. (C-N) 14,3°C und Klp. (N-I) 39,5°C. Die Mutterlaugen wurden nicht aufgearbeitet. Gewünschtenfalls können diese aber wiederum äquilibriert und kristallisiert werden.

In analoger Weise wurden folgende Verbindungen hergestellt:

p-[trans-4-(trans-1-Propenyl)cyclohexyl]benzonitril; Smp. (C-N) 66,3°C, Klp. (N-I) 73,0°C,
p-[trans-4-(trans-1-Butenyl)cyclohexyl]benzonitril; Smp. (C-N) 45,1°C, Klp. (N-I) 51,8°C,
p-[trans-4-(trans-1-Pentenyl)cyclohexyl]benzonitril; Smp. (C-N) 15,6°C, Klp. (N-I) 58,5°C,
p-[trans-4-(trans-1-Heptenyl)cyclohexyl]benzonitril; Smp. (C-N) 17,9°C, Klp. (N-I) 49,2°C.

**Beispiel 8**

In einem Sulfierkolben mit mechanischem Rührer und Thermometer wurde unter Argonbegasung ein Gemisch von 2,75 g p-[trans-4-(erythro-1,2-Dibrompentyl)cyclohexyl]benzonitril und 20 ml Eisessig bei Raumtemperatur mit 2,42 g Zinkpulver versetzt und dann 2 Stunden gerührt, wobei sich das Reaktionsgemisch auf 33°C erwärmte und das Edukt allmählich in Lösung ging. Anschliessend wurde das Reaktionsgemisch auf 100 ml Wasser gegossen und dreimal mit je 100 ml Petroläther extrahiert. Die organischen Phasen wurden

zweimal mit je 100 ml Wasser und einmal mit 50 ml gesättigter Watriumhydrogencarbonat-Lösung gewaschen, über Magnesiumsulfat getrocknet und eingeengt. Hierbei wurden 1,43 g (99%) p-[trans-4-(trans-1-Pentenyl)cyclohexyl]benzonitril in einer Reinheit von 99,5 % erhalten; Smp. (C-N) 15,6° C, Klp. (N-I) 58,5° C.

Das als Ausgangsmaterial verwendete p-[trans-4-(erythro-1,2-Dibrompentyl)cyclohexyl]benzonitril wurde wie folgt hergestellt:

a) In einem Sulfierkolben mit Thermometer, Tropftrichter und mechanischem Rührer wurde unter Argonbegasung ein Gemisch von 1,51 g 90 %-iger m-Chlorperbenzoesäure und 3,0 g gemahlenem Kaliumcarbonat in 60 ml Methylenchlorid bei 0° C vorgelegt und innert 15 Minuten mit einer Lösung von 2,0 g p-[trans-4-(1-Pentenyl)cyclohexyl]benzonitril (hergestellt nach Beispiel 6; trans-1-Pentenyl/cis-1-Pentenyl-Verhältnis ca. 5 : 95) in 20 ml Methylenchlorid versetzt. Anschliessend wurde das Kühlbad entfernt und das Reaktionsgemisch nach insgesamt 75 Minuten und 105 Minuten nochmals mit je 0,75 g 90 %-iger m-Chlorperbenzoesäure versetzt. Das Reaktionsgemisch wurde noch 60 Minuten bei Raumtemperatur gerührt, dann auf 50 ml 10 %-ige (Gew./Vol.) Natriumthiosulfat-Lösung gegossen und dreimal mit je 100 ml Methylenchlorid extrahiert. Die organischen Phasen wurden mit 50 ml gesättigter Natriumhydrogencarbonat-Lösung gewaschen, über Magnesiumsulfat getrocknet und eingeengt. Hierbei wurden 2,1 g (98 %) p-[trans-4-(1,2-Epoxypentyl)cyclohexyl]benzonitril (trans-1,2-Epoxypentyl/cis-1,2-Epoxypentyl-Verhältnis ca. 5 : 95) als farbloses Öl erhalten; Rf-Werte (Essigester/Petroläther Vol. 10 : 90): trans-1,2-Epoxypentyl-Isomer 0,17, cis-1,2-Epoxypentyl-Isomer 0,14.

b) In einem Rundkolben mit Tropftrichter wurde unter Argonbegasung eine Lösung von 2,46 g Triphenylphosphin in 30 ml Methylenchlorid bei 0° C vorgelegt und tropfenweise solange mit einer ca. 1M Lösung von Brom in Methylenchlorid versetzt, bis eine schwach gelbe Farbe bestehen blieb. Anschliessend wurde die Lösung am Rotationsverdampfer vorsichtig eingeengt und am Hochvakuum nachgetrocknet. Der resultierende, kristalline Rückstand wurde in 30 ml Benzol aufgeschlämmt, mit einer Lösung von 2,1 g p-[trans-4-(1,2-Epoxypentyl)cyclohexyl]benzonitril in 10 ml Benzol versetzt und 3 Stunden zum Rückfluss erhitzt. Filtration der warmen Reaktionslösung an Kieselgel mit Toluol ergab 3,0 g kristallines Rohprodukt, welches nach Niederdruckchromatographie (0,5 bar) an Kieselgel mit Hexan/Toluol (Vol. 1 : 1) 2,61 g (81 %) fast reines p-[trans-4-(erythro-1,2-Dibrompentyl)cyclohexyl]benzonitril als farblose Kristalle lieferte. Durch Umkristallisation aus 90 ml Petroläther/Essigester (Vol 2 : 1) wurden schliesslich 2 09 g (65%) sehr reines erythro-Dibromid erhalten; Smp. 140,9° C.

In analoger Weise wurden folgende Verbindungen hergestellt:

p-[trans-4-(trans-1-Propenyl)cyclohexyl]benzonitril; Smp. (C-N) 66,3° C, Klp. (N-I) 73,0° C,
p-[trans-4-(trans-1-Butenyl)cyclohexyl]benzonitril; Smp. (C-N) 45,1° C, Klp. (N-I) 51,8° C,
p-[trans-4-(trans-1-Hexenyl)cyclohexyl]benzonitril; Smp. (C-N) 14,4° C, Klp. (N-I) 39,2° C,
p-[trans-4-(trans-1-Heptenyl)cyclohexyl]benzonitril; Smp. (C-N) 17,9° C, Klp. (N-I) 49,2° C.

**Beispiel 9**

In analoger Weise zu den Beispielen 3, 5, 6 und 8 wurde 4-[2-(p-Cyanophenyl)äthyl]cyclohexanon in trans-4-[2-(p-Cyanophenyl)äthyl]cyclohexancarboxaldehyd übergeführt und dieser zu p-[2-(trans-4-(trans-1-Alkenyl)cyclohexyl)äthyl]benzonitrilenweiter umgesetzt.

Das als Ausgangsmaterial verwendete 4-[2-(p-Cyanophenyl)äthyl]cyclohexanon wurde wie folgt hergsstellt:

a) In einem Sulfierkolben wurden unter Rühren und Stickstoffbegasung 149 g Methoxymethyl-triphenylphosphoniumchlorid und 860 ml t-Butylmethyläther bei Raumtemperatur vorgelegt, die Suspension auf -10° C gekühlt und innert 10 Minuten mit 51,6 g Kalium-t-butylat versetzt. Die Suspension wurde noch 30 Minuten bei -10° C bis 0° C gerührt und dann innert 45 Minuten bei 0° C tropfenweise mit einer Lösung von 47,3 g 4,4-Äthylendioxycyclohexanon in 720 ml Tetrahydrofuran versetzt. Die orange Suspension wurde noch 2 Stunden bei Raumtemperatur weiter gerührt, dann auf 5 l Hexan gegossen, 10 Minuten gerührt und genutscht. Das Filtrat wurde im Vakuum eingeengt und das erhaltene gelb-bräunliche Öl (104,1 g) mit 500 ml Hexan versetzt und genutscht. Das Filtrat wurde im Vakuum eingeengt, wobei 61,7 g gelbbräunliches Öl erhalten wurden. Chromatographische Trennung dieses Rohproduktes an Kieselgel mit Methylenchlorid/Aceton (Vol. 98: 2 und 95 : 5) ergab schliesslich 53,5 g 1,1-Äthylendioxy-4-(methoxymethylen)cyclohexan als farbloses Öl.

b) In einem Rundkolben wurde unter Stickstoffbegasung ein Gemisch von 28,2 g 1.1-Äthylendioxy-4-(methoxymethylen)cyclohexan, 770 ml Eisessig und 385 ml Wasser 1 Stunde zum Rückfluss erhitzt. Danach wurde die gelbliche klare Lösung auf Raumtemperatur gekühlt, mit 800 ml Wasser verdünnt und dreimal mit je 700 ml Methylenchlorid extrahiert. Die organischen Phasen wurden zweimal mit je 500 ml 10 %-iger (Gew./Vol.) Natriumcarbonat-Lösung gewaschen, über Natriumsulfat getrocknet, filtriert und eingeengt. Chromatographische Trennung der erhaltenen bräunlichen Flüssigkeit (18,5 g) an Kieselgel mit Methylenchlorid als Eluens ergab schliesslich 16,7 g 4-Formylcyclohexanon als bräunliche Flüssigkeit.

c) In einem Sulfierkolben wurden unter Rühren und Stickstoffbegasung 63,3 g p-Cyanobenzyltriphenylphosphoniumchlorid, 17,2 g Kalium-t-butylat und 195 ml Äthylenglykoldimethyläther vorgelegt, wobei die Innentemperatur bis auf 44° C anstieg. Die braune Suspension wurde auf 0° C gekühlt und innert 2 Minuten mit einer Lösung von 16,7 g 4-Formyl-cyclohexanon in 100 ml Äthylenglykoldimethyläther versetzt. Danach wurde das Kühlbad entfernt und das Reaktionsgemisch noch 3,5 Stunden bei Raumtemperatur gerührt.

Anschliessend wurde die Suspension auf 500 ml Wasser gegossen und dreimal mit je 600 ml Methylenchlorid extrahiert. Die organischen Phasen wurden zweimal mit je 500 ml 10 %-iger (Gew./Vol.) Kochsalzlösung gewaschen, über Natriumsulfat getrocknet, filtriert und eingeengt, wobei 76,9 g einer bräunlichen Paste zurückblieben. Chromatographische Trennung dieses Rohproduktes an Kieselgel mit Methylenchlorid als Eluens ergab 33,0 g 4-[2-(p-Cyanophenyl)vinyl]cyclohexanon als gelbbräunliches Öl.

d) In einem Rundkolben mit Magnetrührer wurde ein Gemisch von 33,0 g 4-[2-(p-Cyanophenyl)vinyl]cyclohexanon, 520 ml, Toluol, 260 ml Äthanol und 3,2 g Palladium/Kohle (5 %) bei Raumtemperatur vorgelegt und das Gemisch bis zum Stillstand der Wasserstoffaufnahme hydriert. Anschliessend wurde die schwarze Suspension genutscht (Nachwaschen mit Toluol) und das Filtrat im Vakuum eingeengt. Das erhaltene, leicht trübe, gelbliche Öl (34,1 g) wurde an Kieselgel chromatographisch getrennt. Methylenchlorid/Hexan (Vol. 1 : 1), Methylenchlorid/Hexan (Vol. 8 : 2) und Methylenchlorid eluierten 25,6 g gelbliches Öl, welches aus t-Butylmethyläther kristallisiert wurde. Hierbei wurden 22,6 g 4-[2-(p-Cyanophenyl)äthyl]cyclohexanon als farblose Kristalle mit Smp. 62,5-64,3°C erhalten.

Auf diese Weise wurden folgende Verbindungen hergestellt:

p-[2-(trans-4-(trans-1-Propenyl)cyclohexyl)äthyl]benzonitril; Smp. (C-I) 61,3°C, Klp. (N-I) 54,2°C,
p-[2-(trans-4-(trans-1-Butenyl)cyclohexyl)äthyl]benzonitril; Smp. (C-I) 42,6°C, Klp. (N-I) 39,7°C,
p-[2-(trans-4-(trans-1-Pentenyl)cyclohexyl)äthyl]benzonitril; Smp. (C-N) 25,1°C, Klp. (N-I) 47,5°C,
p-[2-(trans-4-(trans-1-Hexenyl)cyclohexyl)äthyl]benzonitril; Smp. (C-N) 16,8°C bzw. 19,7°C (2 Modifikationen), Klp. (N-I) 34,6°C,
p-[2-(trans-4-(trans-1-Heptenyl)cyclohexyl)äthyl]benzonitril; Smp. (C-N) 31,6° C, Klp. (N-I) 43,6°C.

**Beispiel 10**

In einem Sulfierkolben mit mechanischem Rührer wurde unter Argonbegasung eine Suspension von 6,64 g Methyltriphenylphosphoniumbromid in 80 ml t-Butylmethyläther bei -10°C innert 3 Minuten mit 2,12 g festem Kalium-t-butylat versetzt. Das Gemisch wurde noch 1 Stunde bei Raumtemperatur gerührt, dann bei 0°C innert 5 Minuten mit einer Lösung von 3,0 g 3-(trans-4-(p-Cyanophenyl)cyclohexyl)propionaldehyd in 20 ml t-Butylmethyläther versetzt und noch 15 Stunden bei Raumtemperatur gerührt. Anschliessend wurde das Reaktionsgemisch dreimal in Diäthyläther/Wasser verteilt. Die organischen Extrakte wurden zweimal mit Wasser gewaschen, über Magnesiumsulfat getrocknet, filtriert und eingedampft. Zur Abtrennung von Triphenylphosphinoxid wurde der Rückstand in Essigester gelöst und die Lösung mit Petroläther verdünnt, filtriert und eingedampft. Chromatographische Trennung des erhaltenen, hellbraunen Öles (4,38 g) an Kieselgel mit Essigester/Petroläther (Vol. 3 : 97) ergab 2,83 g weisse Kristalle. Umkristallisation aus Methanol und Aufarbeitung der Mutterlauge ergab schliesslich insgesamt 2,116 g p-[trans-4-(3-Butenyl)-cyclohexyl]-benzonitril als weisse Kristalle; Smp. (C-N) 49,5°C, Klp. (N-I) 52,5°C.

Der als Ausgangsmaterial verwendete 3-[trans-4-(p-Cyanophenyl)cyclohexyl]propionaldehyd urde wie folgt hergestellt:

a) In einem Sulfierkolben mit mechanischem Rührer wurde unter Argonbegasung eine Suspension von 29,0 g Methoxymethyl-triphenylphosphoniumchlorid in 200 ml t-Butylmethyläther bei -10°C innert 3 Minuten mit 9,7 g Kalium-t-butylat versetzt. Die orange Suspension wurde noch 1 Stunde bei ca. 0°C gerührt, dann bei -10°C innert 10 Minuten tropfenweise mit einer Lösung von 12,0 g trans-4-(p-Cyanophenyl)cyclohexancarboxaldehyd in 90 ml t-Butylmethyläther versetzt und noch 45 Minuten bei 0°C gerührt. Anschliessend wurde das Reaktionsgemisch dreimal in Diäthyläther/Wasser verteilt. Die organischen Extrakte wurden zweimal mit Wasser gewaschen, über Magnesiumsulfat getrocknet, filtriert und eingedampft. Zur Abtrennung von Triphenylphosphinoxid wurde der Rückstand im Essigester gelöst und die Lösung mit Petroläther verdünnt, filtriert und eingedampft. Chromatographische Trennung des gelblichen, kristallinen Rückstandes (16,3 g) an Kieselgel mit Essigester/Petroläther (Vol. 5 : 95) ergab 10,1 g (74 %) p-[trans-4-(2-Methoxyvinyl)cyclohexyl]benzonitril als weisse Kristalle.

b) Eine Lösung von 10,1 g p-[trans-4-(2-Methoxyvinyl)-cyclohexyl]benzonitril in 200 ml Tetrahydrofuran/2N Salzsäure (Vol. 4 : 1) wurde unter Rühren 1 Stunde zum Rückfluss erhitzt. Anschliessend wurde das Reaktionsgemisch dreimal in Diäthyläther/Wasser verteilt. Die organischen Extrakte wurden zweimal mit Wasser gewaschen, über Magnesiumsulfat getrocknet, filtriert und eingedampft, wobei 9,8 g 2-[trans-4-(p-Cyanophenyl)cyclohexyl]acetaldehyd als leicht gelblicher, kristalliner Rückstand erhalten wurden.

c) In einem Sulfierkolben mit mechanischem Rührer wurde unter Argonbegasung eine Suspension von 22,2 g Methoxymethyl-triphenylphosphoniumchlorid in 150 ml t-Butylmethyläther bei 0°C innert 3 Minuten mit 7,4 g festem Kalium-t-butylat versetzt. Die orange Suspension wurde noch 1 Stunde bei 0°C gerührt und dann innert 10 Minuten tropfenweise mit einer Lösung von 9,8 g 2-[trans-4-(p-Cyanophenyl)-cyclohexyl]acetaldehyd in 100 ml Tetrahydrofuran versetzt. Anschliessend wurde die Suspension unter Rühren langsam auf Raumtemperatur erwärmen gelassen. Nach 15 Stunden wurde die Suspension dreimal in Diäthyläther/Wasser verteilt. Die organischen Extrakte wurden zweimal mit Wasser gewaschen, über Magnesiumsulfat getrocknet, filtriert und eingedampft. Zur Abtrennung von Triphenylphosphinoxid wurde der Rückstand in Essigester gelöst und die Lösung mit Petroläther verdünnt, filtriert und eingedampft. Chromatographische Trennung des zurückbleibenden gelblichen Öles (13,7 g) an Kieselgel mit Essigester/Petroläther (Vol. 5 : 95) ergab 10,5 g (96

%) p-[trans-4-(3-Methoxy-2-propenyl)cyclohexyl]-benzonitril als farbloses Öl.

d) Eine Lösung von 10,5 g p-[trans-4-(3-Methoxy-2-propenyl)-cyclohexyl]benzonitril in 200 ml Tetrahydrofuran/2N Salzsäure (Vol. 4 : 1) wurde unter Rühren 45 Minuten zum Rückfluss erhitzt. Danach wurde das Reaktiongemisch dreimal in Diäthyläther/Wasser verteilt. Die organischen Extrakte wurden zweimal mit Wasser gewaschen, über Magnesiumsulfat getrocknet, filtriert und eingedampft. Chromatographische Trennung des weissen, kristallinen Rückstandes (9,9 g) an Kieselgel mit Essigester/Petroläther (Vol. 10 : 90 und 30 : 70) ergab schliesslich 9,4 g (95 %) 3-[trans-4-(p-Cyanophenyl)cyclohexyl]propionaldehyd als weisse Kristalle.

**Beispiel 11**

In einem Rundkolben mit Magnetrührer wurde unter Argonbegasung eine Lösung von 9,54 g p-[trans-4-(erythro-3,4-Dibrompentyl)cyclohexyl]benzonitril in 100 ml Eisessig mit 9,8 g Zink-Pulver versetzt. Das Gemisch wurde 30 Minuten bei Raumtemperatur gerührt und dann dreimal in Petroläther/Wasser verteilt. Die organischen Extrakte wurden zweimal mit Wasser gewaschen, über Magnesiumsulfat getrocknet, filtriert und eingeengt. Chromatographische Trennung des öligen Rückstandes (5,56 g) an mit Silbernitrat beschichtetem Kieselgel (hergestellt durch Aufschlämmen von Kieselgel in 500 ml einer 0,2M Lösung von Silbernitrat in Acetonitril, anschliessende Filtration und Trocknung des Rückstandes) unter Verwendung von Diäthyläther/Hexan (Vol. 1 : 9) als Eluens ergab 3,2 g Rohprodukt als weisse Kristalle. Nach Umkristallisation aus 80 ml Methanol wurden 1,65 g (28 %) p-[trans-4-(trans-3-Pentenyl)cyclohexyl]benzonitril als weisse Kristalle erhalten. Die Mutterlauge und die unreinen Fraktionen aus der chromatographischen Trennung wurden vereinigt und nochmals an mit Silbernitrat beschichteten Kieselgel unter Verwendung von Diäthyläther/Hexan (Vol. 1 : 9) als Eluens gereinigt. Umkristallisation des erhaltenen, kristallinen Rohproduktes (1,5 g) aus 40 ml Methanol ergab weitere 0,65 g p-[trans-4-(trans-3-Pentenyl)cyclohexyl]benzonitril als weisse Kristalle; Smp. (C-N) 59,8°C. Klp. (N-I) 73,7°C.

Das als Ausgangsmaterial verwendete p-[trans-4-(erythro-3,4-Dibrompentyl)cyclohexyl]benzonitril wurde wie folgt hergestellt:

a) In einem Sulfierkolben mit mechanischem Rührer wurde unter Argonbegasung eine Suspension von 14,8 g Äthyltriphenylphosphoniumbromid in 150 ml t-Butylmethyläther bei -10°C innert 5 Minuten mit 4,54 g festem Kalium-t-butylat versetzt. Die Suspension wurde noch 1 Stunde bei Raumtemperatur gerührt, dann bei 0°C innert 5 Minuten tropfenweise mit einer Lösung von 6,4 g 3-[trans-4-(p-Cyanophenyl)cyclohexyl]propionaldehyd in 40 ml t-Butylmethyläther versetzt und noch 15 Stunden bei Raumtemperatur gerührt. Anschliessend wurde das Reaktionsgemisch dreimal in Diäthyläther/Wasser verteilt. Die organischen Extrakte wurde zweimal mit Wasser gewaschen, über Magnesiumsulfat getrocknet, filtriert und eingeengt. Zur Abtrennung von Triphenylphosphinoxid wurde der Rückstand in Essigester gelöst und die Lösung mit Petroläther verdünnt, filtriert und eingeengt. Chromatographische Trennung des zurückbleibenden gelblichen Öles (8,55 g) an Kieselgel mit Essigester/Petroläther (Vol. 3 : 97) ergab 5,93 g (89 %) p-[trans-4-(3-Pentenyl)cyclohexyl]benzonitril als weisse Kristalle.

b) Eine Lösung von 4,49 g 90 %-iger m-Chlorperbenzoesäure in 100 ml Methylenchlorid wurde mit 11,3 g gemahlenem Kaliumcarbonat versetzt. Das Gemisch wurde bei 0°C innert 5 Minuten tropfenweise mit einer Lösung von 5,93 g p-[trans-4-(3-Pentenyl)cyclohexyl]benzonitril in 20 ml Methylenchlorid versetzt und noch 2 Stunden bei Raumtemperatur gerührt. Anschliessend wurde das Gemisch nochmals mit 4,49 g 90 %-iger m-Chlorperbenzoesäure versetzt und weitergerührt. Nach insgesamt 70 Stunden wurde das Reaktionsgemisch dreimal in Methylenchlorid/10 %-iger Natriumthiosulfat-Lösung verteilt. Die organischen Extrakte wurden mit Natriumthiosulfat-Lösung und Wasser gewaschen, über Magnesiumsulfat getrocknet, filtriert und eingeengt. Chromatographische Trennung des zurückbleibenden, leicht gelblichen Öles (6,3 g) an Kieselgel mit Essigester/Petroläther (Vol. 10 : 90) ergab 6,27 g (99,5 %) p-[trans-4-(3,4-Epoxypentyl)cyclohexyl]benzonitril als farbloses Öl.

c) Eine Lösung von 7,4 g Triphenylphosphin in 80 ml Methylenchlorid wurde bei 0°C unter Argonbegasung tropfenweise mit einer Lösung von 1,5 ml Brom in 20 ml Methylenchlorid versetzt, bis die Gelbfärbung bestehen blieb. Danach wurde das Gemisch am Rotationsverdampfer eingedampft und der Rückstand 1 Stunde am Hochvakuum getrocknet. Der gelbe, kristalline Rückstand wurde in 120 ml Benzol aufgeschlämmt. Die Suspension wurde mit 6,27 g p-[trans-4-(3,4-Epoxypentyl)cyclohexyl]benzonitril versetzt und unter Rühren 1 Stunde zum Rückfluss erhitzt. Anschliessend wurde das Reaktionsgemisch an Kieselgel mit Toluol als Eluens filtriert. Einengen der produkthaltigen Fraktionen ergab schliesslich 9,54 g (99,1 %) p-[trans-4-(erythro-3,4-Dibrompentyl)cyclohexyl]benzonitril als leicht bräunliches Öl.

**Beispiel 12**

Ein Gemisch von 1,00 g 2-(trans-1-Pentenyl)-1,3-propandiol, 1,11 g p-Butyloxybenzaldehyd, 3 Tropfen 2N Schwefelsäure und 40 ml Toluol wurde 2 Stunden unter Wasserabscheidung zum Rückfluss erhitzt.

Anschliessend wurde das Gemisch mit 7 Tropfen Triäthylamin versetzt, abkühlen gelassen, mit 5 ml gesättigter Natriumhydrogencarbonat-Lösung und dreimal mit je 10 ml Wasser gewaschen, über Natriumcarbonat getrocknet, filtriert und eingeengt. Der semi-kristalline Rückstand (1,86 g) wurde an Kieselgel mit Hexan/Diäthyläther (Vol. 97 : 3) chromatographiert. Die produkthaltigen Fraktionen wurden gesammelt (0,99 g) und zweimal bei -25°C aus Hexan umkristallisiert. Es resultierten 0,44 g reines trans-2-(p-Butyloxyphenyl)-5-(trans-1-pentenyl)-m-dioxan; Smp. (C-N) 60,6°C, Klp. (N-I) 61,9°C.

Das als Ausgangsmaterial verwendete

2-(trans-1-Pentenyl)-1,3-propandiol wurde wie folgt hergestellt:

Zu einer Suspension von 5,3 g Lithiumaluminiumhydrid in 200 ml trockenem Tetrahydrofuran wurde unter Rühren in Inertgasatmosphäre innert 1 Stunde bei 5°C eine Lösung von 16,1 g 2-(trans-1-Pentenyl)malonsäurediäthylester (Tetrahedron Lett. 1979, 861) in 75 ml Tetrahydrofuran zugetropft. Das Gemisch wurde noch 3,5 Stunden bei Raumtemperatur gerührt und dann nacheinander tropfenweise mit 15 ml Aceton und 20 ml gesättigter Natriumhydrogencarbonat-Lösung versetzt. Das Reaktionsgemisch wurde filtriert, das Filtrat eingeengt und der Rückstand (8,2 g) im Kugelrohr bei 150°C/ca. 1 Torr destilliert. Hierbei wurden 6,5 g 2-(trans-1-Pentenyl)-1,3-propandiol als farbloses Öl erhalten.

In analoger Weise wurden folgende Verbindungen hergestellt:

p-[trans-5-(trans-1-Propenyl)-m-dioxan-2-yl]benzonitril; Smp. (C-I) 97°C, Klp. (N-I) 73°C,
p-[trans-5-(trans-1-Butenyl)-m-dioxan-2-yl]benzonitril; Smp. (C-I) 92,2°C,
p-[trans-5-(trans-1-Pentenyl)-m-dioxan-2-yl]benzonitril; Smp. (C-I) 67,2°C, Klp. (N-I) 59,1°C,
p-[trans-5-(trans-1-Hexenyl)-m-dioxan-2-yl]benzonitril; Smp. (C-I) 50,5°C, Klp. (N-I) 37,0°C,
p-[trans-5-(trans-1-Heptenyl)-m-dioxan-2-yl]benzonitril; Smp. (C-I) 49,3°C, Klp. (N-I) 49,2°C.

**Patentansprüche** für die Vertragsstaaten BE, CH, DE, FR, GB, IT, LI, NL, SE

1. Verbindungen der allgemeinen Formel

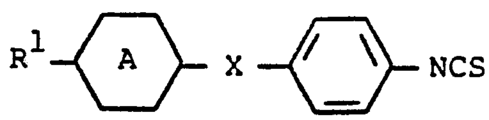

I

worin X eine einfache Kovalenzbindung, die Äthylengruppe $-CH_2CH_2-$, 1,4-Phenylen, einen 2,5-disubstituierten Pyrimidinring oder die Gruppe der Formel

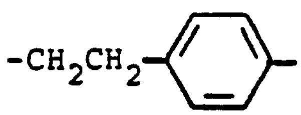

IA

darstellt; Ring A trans-1,4-Cyclohexylen oder, sofern X eine einfache Kovalenzbindung darstellt, auch 1,4-Phenylen, einen 2,5-disubstituierten Pyrimidinring oder einen trans-2,5-disubstituierten m-Dioxanring bezeichnet; und $R^1$ geradkettiges trans-1-Alkenyl mit 2 bis 12 Kohlenstoffatomen oder geradkettiges trans-3-Alkenyl mit 4 bis 12 Kohlenstoffatomen bedeutet oder, sofern X $-CH_2CH_2-$, 1,4-Phenylen, einen 2,5-disubstituierten Pyrimidinring oder eine Gruppe der Formel IA darstellt oder Ring A einen 2,5-disubstituierten Pyrimidinring oder einen trans-2,5-disubstituierten m-Dioxanring bezeichnet, $R^1$ auch geradkettiges Alkyl mit 1 bis 12 Kohlenstoffatomen bedeutet; und einer der Benzolringe gegebenenfalls einen lateralen Fluor-Substituenten aufweist.

2. Verbindungen nach Anspruch 1, dadurch gekennzeichnet, dass X eine einfache Kovalenzbindung, 1,4-Phenylen oder einen 2,5-disubstituierten Pyrimidinring darstellt.

3. Verbindungen der allgemeinen Formel

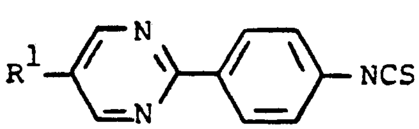

Ia

worin $R^1$ geradkettiges trans-1-Alkenyl mit 2 bis 12 Kohlenstoffatomen, geradkettiges trans-3-Alkenyl mit 4 bis 12 Kohlenstoffatomen oder geradkettiges Alkyl mit 1 bis 12 Kohlenstoffatomen bedeutet.

4. Verbindungen der allgemeinen Formel

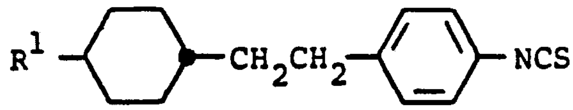

Id

worin $R^1$ geradkettiges trans-1-Alkenyl mit 2 bis 12 Kohlenstoffatomen, geradkettiges trans-3-Alkenyl mit 4 bis 12 Kohlenstoffatomen oder geradkettiges Alkyl mit 1 bis 12 Kohlenstoffatomen bedeutet.

5. Verbindungen der allgemeinen Formel

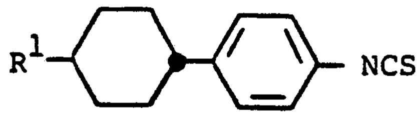

Ib

worin $R^1$ geradkettiges trans-1-Alkenyl mit 2 bis 12 Kohlenstoffatomen oder geradkettiges trans-3-Alkenyl mit 4 bis 12 Kohlenstoffatomen bedeutet.

6. Verbindungen nach einem der Ansprüche 1 bis 5, dadurch gekennzeichnet, dass $R^1$ geradkettiges trans-1-Alkenyl mit 2 bis 7 Kohlenstoffatomen oder geradkettiges trans-3-Alkenyl mit 4 bis 7 Kohlenstoffatomen bedeutet.

7. Verbindungen nach einem der Ansprüche 1 bis 4, dadurch gekennzeichnet, dass $R^1$ geradkettiges Alkyl mit 1 bis 7 Kohlenstoffatomen bedeutet.

8. Flüssigkristallines Gemisch mit mindestens 2 Komponenten, dadurch gekennzeichnet, dass mindestens eine Komponente eine der in Anspruch 1 definierten Verbindungen ist.

9. Verfahren zur Herstellung der Verbindungen der in Anspruch 1 definierten Formel 1, dadurch gekennzeichnet, dass man eine Verbindung der allgemeinen Formel

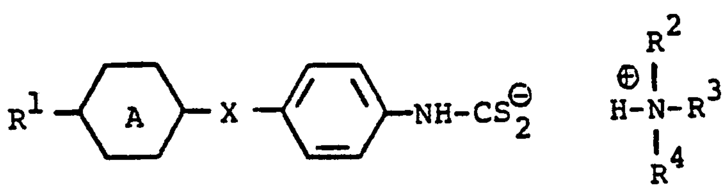

II

worin $R^1$, X und Ring A die in Anspruch 1 gegebenen Bedeutungen haben, einer der Benzolringe gegebenenfalls einen lateralen Fluor-Substituenten aufweist, $R^2$ und $R^3$ $C_1$-$C_7$-Alkyl bezeichnen und $R^4$ Wasserstoff oder $C^1$-$C^7$-Alkyl bedeutet, in Gegenwart eines Amins mit einem Chlorameisensäureester umsetzt.

10. Verwendung der in Anspruch 1 definierten Verbindungen für elektro-optische Zwecke.

**Patentansprüche** für den Vertragsstaat AT

1. Flüssigkristallines Gemisch mit mindestens 2 Komponenten, dadurch gekennzeichnet, dass mindestens eine Komponente eine Verbindung der allgemeinen Formel

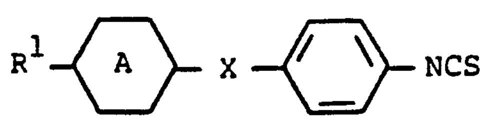

I

worin X eine einfache Kovalenzbindung, die Äthylengruppe $-CH_2CH_2-$, 1,4-Phenylen, einen 2,5-disubstituierten Pyrimidinring oder die Gruppe der Formel

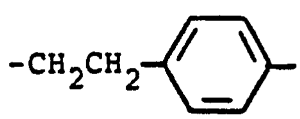

IA

darstellt; Ring A trans-1,4-Cyclohexylen oder, sofern X eine einfache Kovalenzbindung darstellt, auch 1,4-Phenylen, einen 2,5-disubstituierten Pyrimidinring oder einen trans-2,5-disubstituierten m-Dioxanring bezeichnet; und $R^1$ geradkettiges trans-1-Alkenyl mit 2 bis 12 Kohlenstoffatomen oder geradkettiges trans-3-Alkenyl mit 4 bis 12 Kohlenstoffatomen bedeutet oder, sofern X $-CH_2CH_2-$, 1,4-Phenylen, einen 2,5-

disubstituierten Pyrimidinring oder eine Gruppe der Formel IA darstellt oder Ring A einen 2,5-disubstituierten Pyrimidinring oder einen trans-2,5-disubstituierten m-Dioxanring bezeichnet, $R^1$ auch geradkettiges Alkyl mit 1 bis 12 Kohlenstoffatomen bedeutet; und einer der Benzolringe gegebenenfalls einen lateralen Fluor-Substituenten aufweist, ist.

2. Flüssigkristallines Gemisch nach Anspruch 1, dadurch gekennzeichnet, dass X eine einfache Kovalenzbindung, 1,4-Phenylen oder einen 2,5-disubstituierten Pyrimidinring darstellt.

3. Flüssigkristallines Gemisch nach Anspruch 1, dadurch gekennzeichnet, dass es mindestens eine Verbindung der allgemeinen Formel

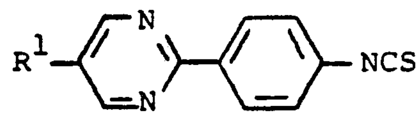
Ia

worin $R^1$ geradkettiges trans-1-Alkenyl mit 2 bis 12 Kohlenstoffatomen, geradkettiges trans-3-Alkenyl mit 4 bis 12 Kohlenstoffatomen oder geradkettiges Alkyl mit 1 bis 12 Kohlenstoffatomen bedeutet, enthält.

4. Flüssigkristallines Gemisch nach Anspruch 1, dadurch gekennzeichnet, dass es mindestens eine Verbindung der allgemeinen Formel

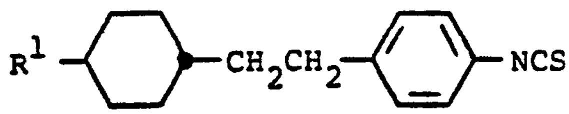
Id

worin $R^1$ geradkettiges trans-1-Alkenyl mit 2 bis 12 Kohlenstoffatomen, geradkettiges trans-3-Alkenyl mit 4 bis 12 Kohlenstoffatomen oder geradkettiges Alkyl mit 1 bis 12 Kohlenstoffatomen bedeutet.

5. Flüssigkristallines Gemisch nach Anspruch 1, dadurch gekennzeichnet, dass es mindestens eine Verbindung der allgemeinen Formel

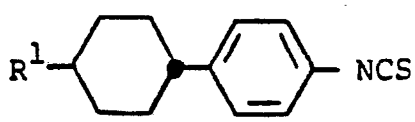
Ib

worin $R^1$ geradkettiges trans-1-Alkenyl mit 2 bis 12 Kohlenstoffatomen oder geradkettiges trans-3-Alkenyl mit 4 bis 12 Kohlenstoffatomen bedeutet.

6. Flüssigkristallines Gemisch nach einem der Ansprüche 1 bis 5, dadurch gekennzeichnet dass $R^1$ geradkettiges trans-1-Alkenyl mit 2 bis 7 Kohlenstoffatomen oder geradkettiges trans-3-Alkenyl mit 4 bis 7 Kohlenstoffatomen bedeutet.

7. Flüssigkristallines Gemisch nach einem der Ansprüche 1 bis 4, dadurch gekennzeichnet, dass $R^1$ geradkettiges Alkyl mit 1 bis 7 Kohlenstoffatomen bedeutet.

8. Verfahren zur Herstellung der Verbindungen der in Anspruch 1 definierten Formel I, dadurch gekennzeichnet, dass man eine Verbindung der allgemeinen Formel

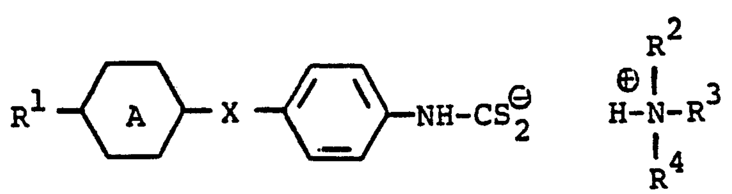
II

worin $R^1$, X und Ring A die in Anspruch 1 gegebenen Bedeutungen haben, einer der Benzolringe gegebenenfalls einen lateralen Fluor-Substituenten aufweist, $R^2$ und $R^3$ $C_1$-$C_7$-Alkyl bezeichnen und $R^4$ Wasserstoff oder $C_1$-$C_7$-Alkyl bedeutet, in Gegenwart eines Amins mit einem Chlorameisensäureester umsetzt.

9. Verwendung der Verbindungen der in Anspruch 1 definierten Formel I für elektro-optische Zwecke.

**Claims** for the Contracting States BE, CH, DE, FR, GB, IT, LI, NL, SE

1. Compounds of the general formula

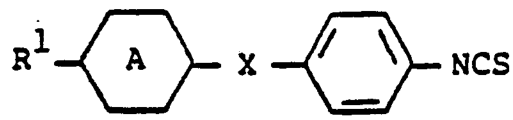

I

wherein X represents a single covalent bond, the ethylene group $-CH_2CH_2-$, 1,4-phenylene, a 2,5-disubstituted pyrimidine ring or the group of the formula

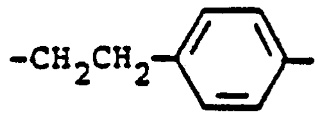

IA;

ring A represents trans-1,4-cyclohexylene or, when X represents a single covalent bond, also 1,4-phenylene, a 2,5-disubstituted pyrimidine ring or a trans-2,5-disubstituted m-dioxane ring; and $R^1$ signifies straight-chain trans-1-alkenyl with 2 to 12 carbon atoms or straight-chain trans-3-alkenyl with 4 to 12 carbon atoms or, when X represents $-CH_2CH_2-$, 1,4-phenylene, a 2,5-disubstituted pyrimidine ring or a group of formula IA or ring A denotes a 2,5-disubstituted pyrimidine ring or a trans-2,5-disubstituted m-dioxane ring, $R^1$ also signifies straight-chain alkyl with 1 to 12 carbon atoms; and one of the benzene rings optionally has a lateral fluorine substituent.

2. Compounds according to claim 1, characterized in that X represents a single covalent bond, 1,4-phenylene or a 2,5-disubstituted pyrimidine ring.

3. Compounds of the general formula

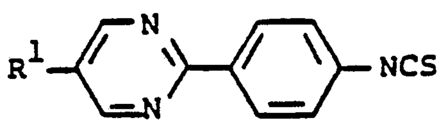

Ia

wherein $R^1$ signifies straight-chain trans-1-alkenyl with 2 to 12 carbon atoms, straight-chain trans-3-alkenyl with 4 to 12 carbon atoms or straight-chain alkyl with 1 to 12 carbon atoms.

4. Compounds of the general formula

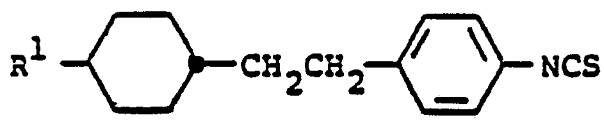

Id

wherein $R^1$ signifies straight-chain trans-1-alkenyl with 2 to 12 carbon atoms, straight-chain trans-3-alkenyl with 4 to 12 carbon atoms or straight-chain alkyl with 1 to 12 carbon atoms.

5. Compounds of the general formula

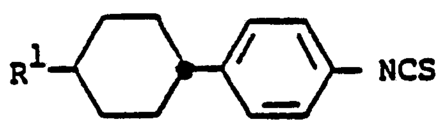

Ib

wherein $R^1$ signifies straight-chain trans-1-alkenyl with 2 to 12 carbon atoms or straight-chain trans-3-alkenyl with 4 to 12 carbon atoms.

6. Compounds according to any one of claims 1 to 5, characterized in that $R^1$ signifies straight-chain trans-1-alkenyl with 2 to 7 carbon atoms or straight-chain trans-3-alkenyl with 4 to 7 carbon atoms.

7. Compounds according to any one of claims 1 to 4, characterized in that $R^1$ signifies straight-chain alkyl with 1 to 7 carbon atoms.

8. A liquid crystalline mixture with at least 2 components, characterized in that at least one component is one of the compounds defined in claim 1.

9. A process for the manufacture of the compounds of formula 1 defined in claim 1, characterized by reacting a compound of the general formula

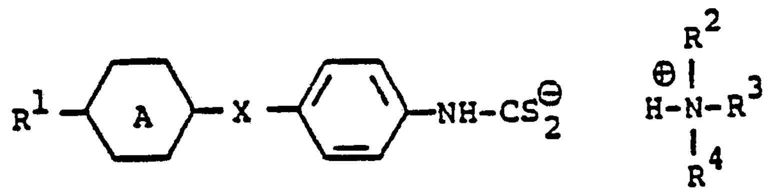

II

wherein $R^1$, X and ring A have the significances given in claim 1, one of the benzene rings optionally has a lateral fluorine substituent, $R^2$ and $R^3$ denote $C_1$-$C_7$-alkyl and $R^4$ signifies hydrogen or $C_1$-$C_7$-alkyl, in the presence of an amine with a chloroformic acid ester.

10. The use of the compounds defined in claim 1 for electro-optical purposes.

**Claims** for the Contracting State AT

1. A liquid crystalline mixture with at least 2 components, characterized in that at least one component is a compound of the general formula

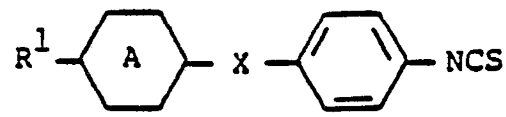

I

wherein X represents a single covalent bond, the ethylene group $-CH_2CH_2-$, 1,4-phenylene, a 2,5-disubstituted pyrimidine ring or the group of the formula

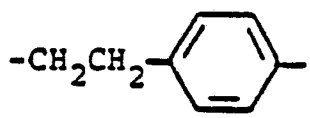

IA;

ring A represents trans-1,4-cyclohexylene or, when X represents a single covalent bond, also 1,4-phenylene, a 2,5-disubstituted pyrimidine ring or a trans-2,5-disubstituted m-dioxane ring: and $R^1$ signifies straight-chain trans-1-alkenyl with 2 to 12 carbon atoms or straight-chain trans-3-alkenyl with 4 to 12 carbon atoms or, when X represents $-CH_2CH_2-$, 1,4-phenylene, a 2,5-disubstituted pyrimidine ring or a group of formula IA or ring A denotes a 2,5-disubstituted pyrimidine ring or a trans-2,5-disubstituted m-dioxane ring, $R^1$ also signifies straight-chain alkyl with 1 to 12 carbon atoms; and one of the benzene rings optionally has a lateral fluorine substituent.

2. A liquid crystalline mixture according to claim 1, characterized in that X represents a single covalent bond, 1,4-phenylene or a 2,5-disubstituted pyrimidine ring.

3. A liquid crystalline mixture according to claim 1, characterized in that it contains at least one compound of the general formula

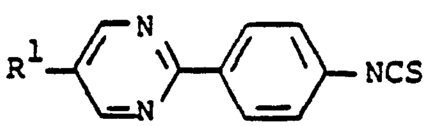

Ia

wherein $R^1$ signifies straight-chain trans-1-alkenyl with 2 to 12 carbon atoms, straight-chain trans-3-alkenyl with 4 to 12 carbon atoms or straight-chain alkyl with 1 to 12 carbon atoms.

4. A liquid crystalline mixture according to claim 1, characterized in that it contains at least one compound of the general formula

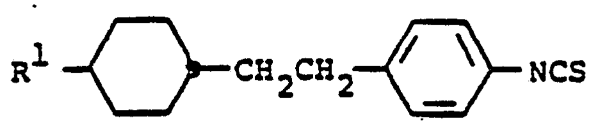

Id

wherein $R^1$ signifies straight-chain trans-1-alkenyl with 2 to 12 carbon atoms, straight-chain trans-3-alkenyl with 4 to 12 carbon atoms or straight-chain alkyl with 1 to 12 carbon atoms.

5. A liquid crystalline mixture according to claim 1, characterized in that it contains at least one compound of

the general formula

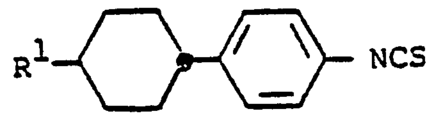

Ib

wherein $R^1$ signifies straight-chain trans-1-alkenyl with 2 to 12 carbon atoms or straight-chain trans-3-alkenyl with 4 to 12 carbon atoms.

6. A liquid crystalline mixture according to any one of claims 1 to 5, characterized in that $R^1$ signifies straight-chain trans-1-alkenyl with 2 to 7 carbon atoms or straight-chain trans-3-alkenyl with 4 to 7 carbon atoms.

7. A liquid crystalline mixture according to any one of claims 1 to 4, characterized in that $R^1$ signifies straight-chain alkyl with 1 to 7 carbon atoms.

8. A process for the manufacture of the compounds of formula 1 defined in claim 1, characterized by reacting a compound of the general formula

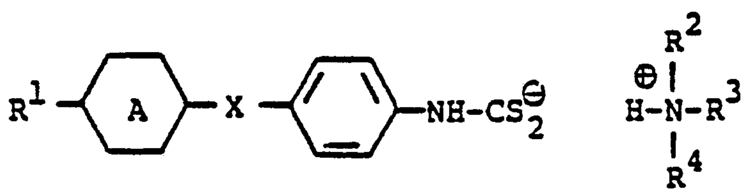

II

wherein $R^1$, X and ring A have the significances given in claim 1, one of the benzene rings optionally has a lateral fluorine substituent, $R^2$ and $R^3$ denote $C_1$-$C_7$-alkyl and $R^4$ signifies hydrogen or $C_1$-$C_7$-alkyl, in the presence of an amine with a chloroformic acid ester.

9. The use of the compounds defined in claim 1 for electro-optical purposes.

**Revendications** pour les etats contractants BE, CH, DE, FR, GB, IT, LI, NL, SE

1. Composés de formule générale

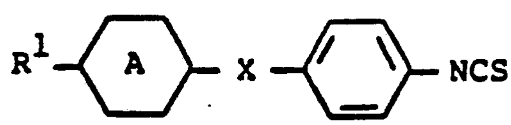

I

dans laquelle X représente une liaison covalente simple, le groupe éthylène $-CH_2CH_2-$, le 1,4-phénylène, un noyau pyrimidine 2,5-disubstitué ou le qroupe de formule

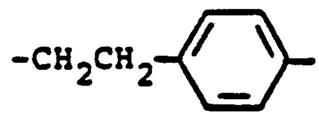

IA

le noyau A représente le trans-1,4-cyclohexylène ou, dans la mesure où X représente une liaison covalente simple, encore un 1,4-phénylène, un noyau pyrimidine 2,5-disubstitué ou un noyau m-dioxanne trans-2,5-disubstitué; et $R^1$ représente un trans-1-alcényle à chaîne droite en C2 à C12 ou un trans-3-alcényle à chaîne droite en C4 à C12, ou encore, dans la mesure où X représente $-CH_2CH_2$ -, le 1,4-phénylène, un noyau pyrimidine 2,5-disubstitué ou un groupe de formule IA ou le noyau A représente un noyau pyrimidine 2,5-disubstitué ou un noyau m-dioxanne trans-2,5-disubstitué, R1 représente également un alcoyle à chaîne droite en C1 à C12; et l'un des noyaux benzène présente éventuellement un substituant fluor latéral.

2. Composés selon la revendication 1, caractérisé en ce que X représente une liaison covalente simple, un 1,4-phénylène ou un noyau pyrimidine 2,5-disubstitué.

3. Composés de formule générale

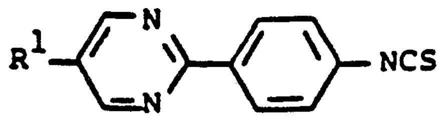

Ia

dans laquelle R1 représente un trans-1-alcényle à chaîne droite en C2 à C12, un trans-3-alcényle à chaîne droite en C4 à C12 ou un alcoyle à chaîne droite en C1 à C12.

4. Composés de formule générale

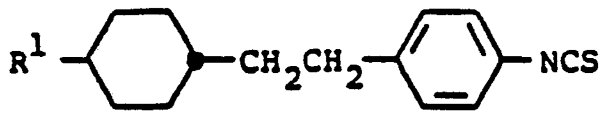

Id

dans laquelle R1 représente un trans-1-alcényle à chaîne droite en C2 à C12, un trans-3-alcényle à chaîne droite en C4 à C12 ou un alcoyle à chaîne droite en C1 à C12.

5. Composés de formule générale

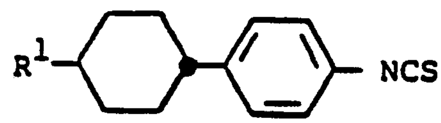

Ib

dans laquelle R1 représente un trans-1-alcényle à chaîne droite en C2 à C12 ou un trans-3-alcényle à chaîne droite en C4 à C12.

6. Composés selon l'une des revendications 1 à 5, caractérisés en ce que R1 représente un trans-1-alcényle à chaîne droite en C2 à C7 ou un trans-3-alcényle à chaîne droite en C4 à C7.

7. Composés selon l'une des revendications 1 à 4, caractérisés en ce que R1 représente un alcoyle à chaîne droite en C1 à C7.

8. Mélange à cristaux liquides ayant au moins 2 composants, caractérisé en ce qu'au moins un composant est un des composés définis dans la revendication 1.

9. Procédé de préparation des composés de formule I définie dans la revendication 1, caractérisé en ce qu'on fait réagir un composé de formule générale

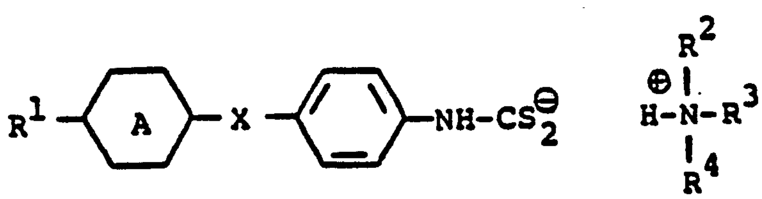

II

dans laquelle R1, X et le noyau A ont les significations données dans la revendication 1, l'un des noyaux benzène présente éventuellement un substituant fluor latéral, R2 et R3 représentent un alcoyle en C1 à C7 et R4 représente un hydrogène ou un alcoyle en C1 à C7, en présence d'une amine avec un ester d'acide chloroformique.

10. Application des composés définis dans la revendication 1 pour des buts électro-optiques.

**Revendications** pour l'etat contractant AT

1. Mélange à cristaux liquides ayant au moins 2 composants, caractérisé en ce qu'au moins un composant est un composé de formule générale

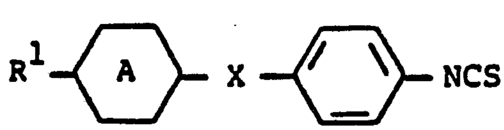

I

dans laquelle X représente une liaison covalente simple, le groupe éthylène $-CH_2CH_2-$, le 1,4-phénylène, un noyau pyrimidine 2,5-disubstitué ou le groupe de formule

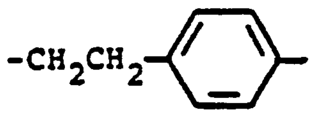 IA

le noyau A représente le trans-1,4-cyclohexylène ou encore, dans la mesure où X représente une liaison covalente simple, le 1,4-phénylène, un noyau pyrimidine 2,5-disubstitué ou un noyau m-dioxanne trans-2,5-disubstitué; et R1 représente un trans-1-alcényle à chaîne droite en C2 à C12 ou un trans-3-alcényle à chaîne droite en C4 à C12, ou encore, dans la mesure où X représente $-CH_2CH_2$ -, le 1,4-phénylène, un noyau pyrimidine 2,5-disubstitué ou un groupe de formule IA ou le noyau A représente un noyau pyrimidine 2,5-disubstitué ou un noyau m-dioxanne trans-2,5-disubstitué, R1 représente également un alcoyle à chaîne droite en C1 à C12; et l'un des noyaux benzène présente éventuellement un substituant fluor latéral.

2. Mélange à cristaux liquides selon la revendication 1, caractérisé en ce que X représente une liaison covalente simple, le 1,4-phénylène ou un noyau pyrimidine 2,5-disubstitué.

3. Mélange à cristaux liquides selon la revendication 1, caractérisé en ce qu'il contient au moins un composé de formule générale

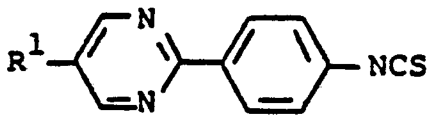 Ia

dans laquelle R1 représente un trans-1-alcényle à chaîne droite en C2 à C12, un trans-3-alcényle à chaîne droite en C4 à C12 ou un alcoyle à chaîne droite en C1 à C12.

4. Mélange à cristaux liquides selon la revendication 1, caractérisé en ce qu'il contient au moins un composé de formule générale

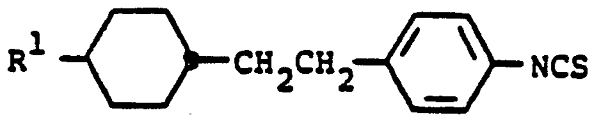 Id

dans laquelle R1 représente un trans-alcényle à chaîne droite en C2 à C12, un trans-3-alcényle à chaîne droite en C4 à C12 ou un alcoyle à chaîne droite en C1 à C12.

5. Mélange à cristaux liquides selon la revendication 1, caractérisé en ce qu'il contient au moins un composé de formule générale

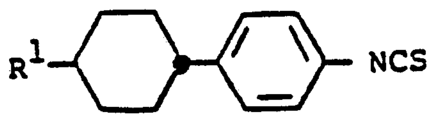 Ib

dans laquelle R1 représente un trans-1-alcényle en C2 à C12 ou un trans-3-alcényle à chaîne droite en C4 à C12.

6. Mélange à cristaux liquides selon l'une des revendications 1 à 5, caractérisé en ce que R1 représente un trans-1-alcényle à chaîne droite en C2 à C7 ou un trans-3-alcényle à chaîne droite en C4 à C7.

7. Mélange à cristaux liquides selon l'une des revendications 1 à 4, caractérisé en ce que R1 représente un alcoyle à chaîne droite en C1 à C7.

8. Procédé de préparation des composés de formule I définie dans la revendication 1, caractérisé en ce qu'on fait réagir un composé de formule générale

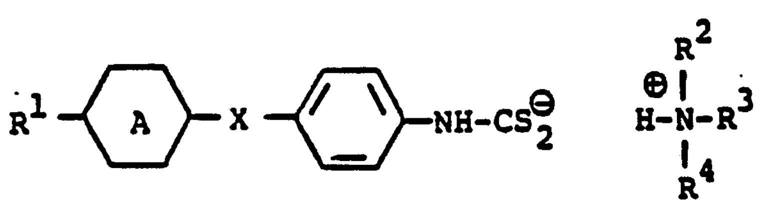 II

dans laquelle R1, X et le noyau A ont les significations données dans la revendication 1, l'un des noyaux benzène présente éventuellement un substituant fluor latéral, R2 et R3 représentent un alcoyle en C1 à C7 et R4 représente un hydrogène ou un alcoyle en C1 à C7, en présence d'une amine avec un ester de l'acide chloroformique.

9. Application des composés de formule I définie dans la revendication 1 pour des buts électro-optiques.